# EUROPÄISCHE PATENTSCHRIFT

(11) **EP 1 651 644 B1**
(45) Veröffentlichungstag und Bekanntmachung des Hinweises auf die Patenterteilung: **22.11.2006**
(21) Anmeldenummer: 04741034.5
(22) Anmeldetag: 15.07.2004
(51) Int. Cl.: C07D 417/12, C07D 417/14, A61K 31/426, A61P 3/10

(54) **SUBSTITUIERTE THIAZOL-BENZOISOTHIAZOLDIOXIDDERIVATIVE, VERFAHREN ZU DEREN HERSTELLUNG UND DEREN VERWENDUNG**
SUBSTITUTED THIAZOLE-BENZOISOTHIAZOLE DIOXIDE DERIVATIVES, METHOD FOR THE PRODUCTION THEREOF AND USE OF THE SAME
DERIVES DE DIOXYDE DE THIAZOL-BENZOISOTHIAZOL SUBSTITUES, PROCEDES POUR LEUR PRODUCTION ET LEUR UTILISATION

(30) Priorität: 28.07.2003 DE 10334309
(43) Veröffentlichungstag der Anmeldung: 03.05.2006
(73) Patentinhaber: Sanofi-Aventis Deutschland GmbH, 65929 Frankfurt am Main (DE)
(72) Erfinder: PETRY, Stefan, 65779 Kelkheim (DE); BARINGHAUS, Karl-Heinz, 61200 Wölfersheim (DE); TENNAGELS, Norbert, 65931 Frankfurt (DE); MUELLER, Guenter, 65843 Sulzbach (DE)
(86) Internationale Anmeldenummer: PCT/EP2004/007847
(87) Internationale Veröffentlichungsnummer: WO 2005/012295

(56) Entgegenhaltungen:
- WO-A-00/76501
- WO-A-92/05164
- US-A- 3 539 584
- US-A1- 2002 055 523

## Beschreibung

Die Erfindung betrifft substituierte Thiazol-Benzoisothiazoldioxidderivate sowie deren physiologisch verträgliche Salze und physiologisch funktionelle Derivate.

Es sind bereits strukturähnliche Benzoisothiazoldioxidderivate im Stand der Technik sowie deren Verwendung zur Behandlung von Diabetes beschrieben (WO 02/11722).

Der Erfindung lag die Aufgabe zugrunde, Verbindungen zur Verfügung zu stellen, mit denen eine Prävention und Behandlung von Diabetes Mellitus möglich ist. Die Verbindungen sollten dazu eine therapeutisch verwertbare Blutzucker senkende Wirkung entfalten. Insbesonders sollten die Verbindungen gegenüber den Verbindungen aus WO 02/11722 eine verbesserte Wirkung oder ein verbessertes ADME-Profil (Absorption, Distribution, Metabolism and Excretion) aufweisen.

Die Erfindung betrifft daher Verbindungen der Formel I, 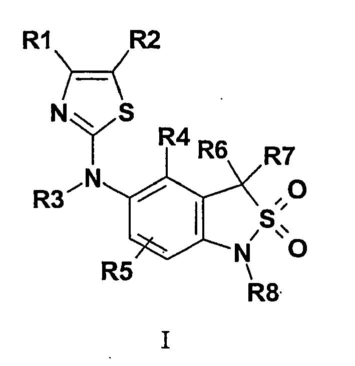 worin bedeuten
- R1, R2: unabhängig voneinander H, Aryl, COOH, (C₁-C₆)-Alkylen-COOH, -COO(C₁₋C₆)-Alkyl, (C₁-C₆)-Alkylen-COO(C₁-C₆)-Alkyl, (C₁C₆)-Akyl, (C₂-C₆)-Alkenyl, (C₁-C₆)-alkylen-Aryl, Heterocyclus, (C₁-C₆)-alkylen-Heterocyclus, CF₃, OCF₃, CN, (CH₂)₁₋₆-OH, O-(C₁-C₆)-Alkyl, CO-(C₁-C₆)-Alkyl, -C(O)O-Alkyl, COOH, CON(R9)(R10) wobei die Arylreste sowie die heterocyclischen Reste ein oder mehrfach substituiert sein können mit F, Cl, Br, (CH₂)₀₋₂OH, (C₁-C₆)-Alkyl, (C₂₋C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆-Alkylen)-Piperazinon, Morpholin, Thiomorpholin, NO₂, CN, O-(C₁-C₆)-Alkyl, S(O)₀₋₂-(C₁-C₆)-Alkyl, SO₂-N(R9)(R10), CO-(C₁-C₆)-Alkyl, -COOH, (C₁-C₆)-Alkylen-COOH, COO(C₁₋C₆)-Alkyl, (C₁-C₆)-Alkylen-COO(C₁-C₆)-Alkyl, (C₃-C₁₀)-Cycloalkyl, Phenyl, wobei diese Piperidinon, Piperazin, Piperazinon, N-(C₁-C₆-Alkylen)-Piperazin, N-(C₁-C₆-Alkylen)-Piperazinbn, Morpholin, Thiomorpholin und Phenylringe ein oder mehrfach substituiert sein können mit F, Cl, Br, (CH₂)₀₋₂OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁-C₆)-Alkyl, CF₃, OCF₃, N(R9)(R10);
- R3: H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl, wobei die Aryl- und Heterocyclischen Reste ein oder mehrfach mit F, Cl, Br, (C₁-C₆)-Alkyl, COOH, COO(C₁-C₆)-Akyl, CF₃ oder OCF₃ substituiert sein können;
- R4, R5: unabhängig voneinander H, F, Cl, Br, (C₁-C₆)-Alkyl, CF₃, OCF₃, NO₂, N(R9)(R10), CN, O-(C₁-C₆)-Alkyl, CO-(C₁-C₆)-Alkyl, COOH , (C₁-C₆)-Alkylen-COOH, CON(R9)(R10), (C₁-C₆)-Alkylen-CON(R9)(R10), COO(C₁-C₆)-Alkyl, (C₁-C₆)-Alkylen-COO(C₁-C₆)-Alkyl, S(O)₀₋₂-(C₁-C₆)-Alkyl, S(O)₂-N(R9)(R10), CH₂OH, CH₂OCH₃;
- R6, R7: unabhängig voneinander H, F, Cl, Br, (C₁-C₆)-Alkyl, Cyclopropyl, Tetrafluorocyclopropyl, Difluorcyclopropyl; oder
- R6 und R7: bilden gemeinsam die Gruppe =CH₂;
- R 8: H, CH₃, CF₃, CH₂OH;
- R 9: H, (C₁-C₄)-Alkyl;
- R 10: H, (C₁-C₄)-Alkyl; oder
- R 9 und R10: bilden gemeinsam mit dem N-Atom, an das sie gebunden sind, ein 3-9 gliedriges Ringsystem;
sowie deren physiologisch verträgliche Salze.

Bevorzugt sind Verbindungen der Formel I worin ein oder mehrere Reste folgende Bedeutung haben:
- R1: Aryl, (C₁-C₆)-Alkyl, (C₂-C₆)-Alkenyl, (C₁-C₆)-alkylen-Aryl, Heterocyclus, (C₁₋C₆)-alkylen-Heterocyclus, CF₃, OCF₃, CN, (CH₂)₁₋₆-OH, O-(C₁-C₆)-Alkyl, CO-(C₁-C₆)-Alkyl, C(O)O-alkyl, COOH, CON(R9)(R10), wobei die Arylreste sowie die heterocyclischen Reste ein oder mehrfach substituiert sein können mit F, Cl, Br, (CH₂)₀₋₂OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆-Alkylen)-Piperazinon, Morpholin, Thiomorpholin, NO₂, CN, O-(C₁₋C₆)-Alkyl, S(O)₀₋₂-(C₁-C₆)-Alkyl, SO₂-N(R9)(R10), CO-(C₁-C₆)-Alkyl, -COOH, (C₁-C₆)-Alkylen-COOH, -COO(C₁-C₆)-Alkyl, (C₀-C₆)-Alkylen -COO(C₁-C₆)-Alkyl, C₃-C₁₀-Cycloalkyl, Phenyl, wobei diese Piperidinon, Piperazin, Piperazinon, N-(C₁-C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Akyl, -(C₁-C₆)-Alkyl, CF₃, OCF₃, N(R9)(R10);
- R2: H, Aryl, COOH, (C₁-C₆)-Alkylen-COOH, -COO(C₁-C₆)-Alkyl, (C₁-C₆)-Alkylen-COO(C₁-C₆)-Alkyl; (C₁-C₆)-Alkyl, (C₂-C₆)-Alkenyl, (C₁-C₆)-alkylen-Aryl, Heterocyclus, (C₁-C₆)-alkylen-Heterocyclus, CF₃, OCF₃, CN, -(CH₂)₁₋₆-OH, O-(C₁-C₆)-Alkyl, CO-(C₁-C₆)-Alkyl, C(O)O-alkyl, COOH, CON(R9)(R10), wobei die Arylreste sowie die heterocyclischen Reste ein oder mehrfach substituiert sein können mit F, Cl, Br, (CH₂)₀₋₂OH, (C₁-C₆)-Akyl, (C₂-C₆)-Alkenyl, (C₂-C₆)-Alkinyl, CF₃, OCF₃, N(R9)(R10), Piperidinon, Piperazin, Piperazinon, N-(C₁-C₆₋Alkylen)-Piperazin, N-(C₁-C₆-Alkylen)-Piperazinon, Morpholin, Thiomorpholin, NO₂, CN, O-(C₁-C₆)-Alkyl" S(O)₀₋₂-(C₁-C₆)-Alkyl, SO₂-N(R9)(R10), CO-(C₁₋C₆)-Alkyl, -COOH, (C₁-C₆)-Alkylen-COOH, -COO(C₁-C₆)-Alkyl, (C₁-C₆)-Alkylen-COO(C₁-C₆)-Alkyl, C₃-C₁₀-Cycloalkyl, Phenyl;
- R3: H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl;
- R4, R5: unabhängig voneinander H, F, Cl, Br, (C₁-C₆)-Alkyl, CF₃, OCF₃, NO₂, N(R9)(R10), CN, O-(C₁-C₆)-Alkyl, CO-(C₁-C₆)-Alkyl, COOH, (C₁-C₆)-Alkylen-COOH, -CON(R9)(R10), (C₁-C₆)-Alkylen-CON(R9)(R10), COO(C₁-C₆)-Alkyl, (C₁-C₆)-Alkylen-COO(C₁-C₆)-Alkyl, S(O)₀₋₂-(C₁-C₆)-Alkyl, S(O)₂-N(R9)(R10), CH₂OH, CH₂OCH₃;
- R6, R7: unabhängig voneinander H, F, Cl, Br, (C₁-C₆)-Alkyl, Cyclopropyl, Tetrafluorocyclopropyl, Difluorcyclopiopyl; oder
- R6 und R7: bilden gemeinsam die Gruppe =CH₂;
- R 8: H, CH₃, CF₃, CH₂OH;
- R 9: H, (C₁-C₄)-Alkyl;
- R10: H, (C₁-C₄)-Alkyl; oder
- R 9 und R10: bilden gemeinsam mit dem N-Atom, an das sie gebunden sind, ein 3-9 gliedriges Ringsystem;
sowie deren physiologisch verträgliche Salze.

Besonders bevorzugt sind Verbindungen der Formel I worin ein oder mehrere Reste folgende Bedeutung haben:
- R1: Phenyl, Naphthyl, Thionaphthyl, Pyridyl, wobei Phenyl, Naphthyl, Thionaphthyl und Pyridyl ein oder mehrfach substituiert sein können mit F, Cl, Br, (CH₂)₀₋₂ OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆₋Alkylen)-Piperazinon, Morpholin, Thiomorpholin, NO₂, CN, O-(C₁-C₆)-Alkyl, S(O)₀₋₂-(C₁-C₆)-Alkyl, SO₂-N(R9)(R10), CO-(C₁-C₆)-Alkyl, COOH, (C₁-C₆)-Alkylen-COOH, COO(C₁-C₆)-Alkyl, (C₁-C₆)-Alkylen-COO(C₁-C₆)-Alkyl, C₃₋C₁₀-Cycloalkyl, Phenyl, wobei diese Piperidinon, Piperazin, Piperazinon, N-(C₁₋C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁₋C₆)-Alkyl, CF₃, OCF₃, N(R9)(R10);
- R2: H, (C₁-C₆)-Alkyl, COOH, (C₁-C₆)-Alkylen-COOH, -COO(C₁-C₆)-Alkyl, (C₁₋C₆)-Alkylen-COO(C₁-C₆)-Alkyl;
- R3: H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl;
- R4, R5: H;
- R6, R7: H;
- R8: H;
- R9: H, (C₁-C₄)-Alkyl;
- R10: H, (C₁-C₄)-Alkyl;
sowie deren physiologisch verträgliche Salze.

Ganz besonders bevorzugt sind Verbindungen der Formel I worin ein oder mehrere Reste folgende Bedeutung haben:
- R1: Phenyl, wobei Phenyl ein oder mehrfach substituiert sein kann mit F, Cl, Br, (CH₂)₀₋₂OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆-Alkylen)-Piperazinon, Morpholin, Thiomorpholin, NO₂, CN, O-(C₁₋C₆)-Alkyl, S(O)₀₋₂-(C₁-C₆)-Alkyl, SO₂-N(R9)(R10), CO-(C₁-C₆)-Alkyl, COOH, (C₁-C₆)-Alkylen-COOH, COO(C₁-C₆)-Alkyl, (C₁-C₆)-Alkylen-COO(C₁-C₆)-Alkyl, C₃-C₁₀-Cycloalkyl, Phenyl, wobei diese Piperidinon, Piperazin, Piperazinon, N-(C₁-C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂-OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁-C₆)-Alkyl, CF₃, OCF₃, N(R9)(R10);
- R2: H, (C₁-C₆)-Alkyl, -C(O)O-(C₁-C₆)-Alkyl, -(C₁-C₆)-Alkylen-C(O)O-(C₁-C₆)-Alkyl, -COOH, -(C₁-C₆)-Alkylen-COOH;
- R3: H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl;
- R4, R5: H;
- R6, R7: H;
- R8: H;
- R9: H;
- R10: H
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B, den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.
Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, ) CON[(C₁-C₆)Alkyl]₂, Cycloalkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ₋Heterocyclus, SO₂-(C₁-C₆)-Alkyl, SO₂-(CH₂)ₙ-Aryl, SO₂-(CH₂)ₙ-Heterocyclus , SO₂₋NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁₋C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heteroeyelus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁₋C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁₋C₆)-Alkyl), N(Aryl)-CO-NH-Aryl, N(Heteroeyelus)-CO-NH-Aryl, N(Aryl)-CO-N-(C₁-C₆)-Akyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.
Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ₋Heterocyclus, SO₂-(C₁-C₆)-Akyl, SO₂-(CH₂)ₙ-Aryl, SO₂-(CH₂)ₙ-Heterocyclus , SO₂₋NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁₋C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Akyl -CO-Heterocyclus, N(C₁-C₆)-Alkyl -COO-Aryl, N(C₁-C₆)-Alkyl - COO-Heterocyclus, N(C₁-C₆)-Akyl -CO-NH-(C₁-C₆)-Akyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Akyl)-Heterocyclus, N((C₁-C₆)-Akyl)-CO-N-(Aryl)₂, N((C₁-C₆)-Alkyl)-CO-N-(Heterocyclus)₂, N(Aryl)-CO-(C₁₋C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁₋C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Akyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ₋Heterocyclus, SO₂-(C₁-C₆)-Alkyl, SO₂-(CH₂)ₙ-Aryl, SO₂-(CH₂)ₙ-Heterocyclus , SO₂₋NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁₋C₆)-Alkyl)(CH₂)ₙ-Heterocyclus, SO₂-N((CH₂)ₙ-Aryl)₂,, SO₂-N((CH₂)ₙ-(Heterocyclus)₂ wobei n =0-6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁₋C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁₋C₆)-Alkyl), N(Aryl)-CO-NH-Aryl, N(Heteroeyelus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Hetetocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁₋C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ₋Heterocyclus, SO₂-(C₁-C₆)-Akyl, SO₂-(CH₂)ₙ-Aryl, SO₂-(CH₂)ₙ-Heterocyclus , SO₂₋NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁₋C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Akyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Akyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁₋C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁₋C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylrestereste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁₋C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ₋Heterocyclus, SO₂-(C₁-C₆)-Alkyl, SO₂-(CH₂)ₙ-Aryl, SO₂-(CH₂)ₙ-Heterocyclus , SO₂₋NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁₋C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁₋C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁₋C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Akyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter Heterocyclus bzw. Heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist.

Geeignete "Heterocyclische Ringe" bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiäzinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyrazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterozyklen.

Die Heterocyclischen Ringe bzw. Heterocyclische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁₋C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ₋Heterocyclus, SO₂-(C₁-C₆)-Alkyl, SO₂-(CH₂)ₙ-Aryl, SO₂-(CH₂)ₙ-Heterocyclus , SO₂₋NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁₋C₆)-Alkyl)(CH₂)ₙ-Heterocyclus, SO₂-N((CH₂)ₙ-Aryl)₂,, SO₂-N((CH₂)ₙ-(Heterocyclus)₂ wobei n =0-6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl-CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Akyl), N(C₁-C₆)-Akyl-CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁₋C₆)-Alkyl, N(Heterocyclus)-CO-(C₁-C₆)-Alkyl, N(Aryl)-COO-(C₁-C₆)-Alkyl, N(Heterocyclus)-COO-(C₁C₆)-Alkyl, N(Aryl)-CO-Aryl, N(Heteroeyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁₋C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Kilogramm Körpergewicht pro Tag, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Ö1-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindung(en) der Formel (I) können auch in Kombination mit weiteren Wirkstoffen verabreicht werden.
Als weitere Wirkstoffe für die Kombinationspräparate sind geeignet:
Alle Antidiabetika, die in der Roten Liste 2003, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen, die in WO 97/26265, WO 99/03861, WO 01/04156, WO 00/34331, WO00/34332, WO91/11457 und US 6,380,357 offenbart wurden, sowie oral wirksame hypoglykämische Wirkstoffe.
Die oral wirksamen hypoglykämischen Wirkstoffe umfassen vorzugsweise Sulphonylfhamstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US00/11833, PCT/US 11490, DE10142734.4 beschrieben verabreicht.

Bei einer Ausfiihrungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo- 2,3,3a,4,6,7-hexahydro-pyrazolo[4,3-c]pyridin-5-yl)-1-(4-chloro-phenyl)-2-oxo-ethyl]- amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1 -(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2- carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi,
Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff ein Blutdrucksenker, wie z.B. ein ACE-Hemmer.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

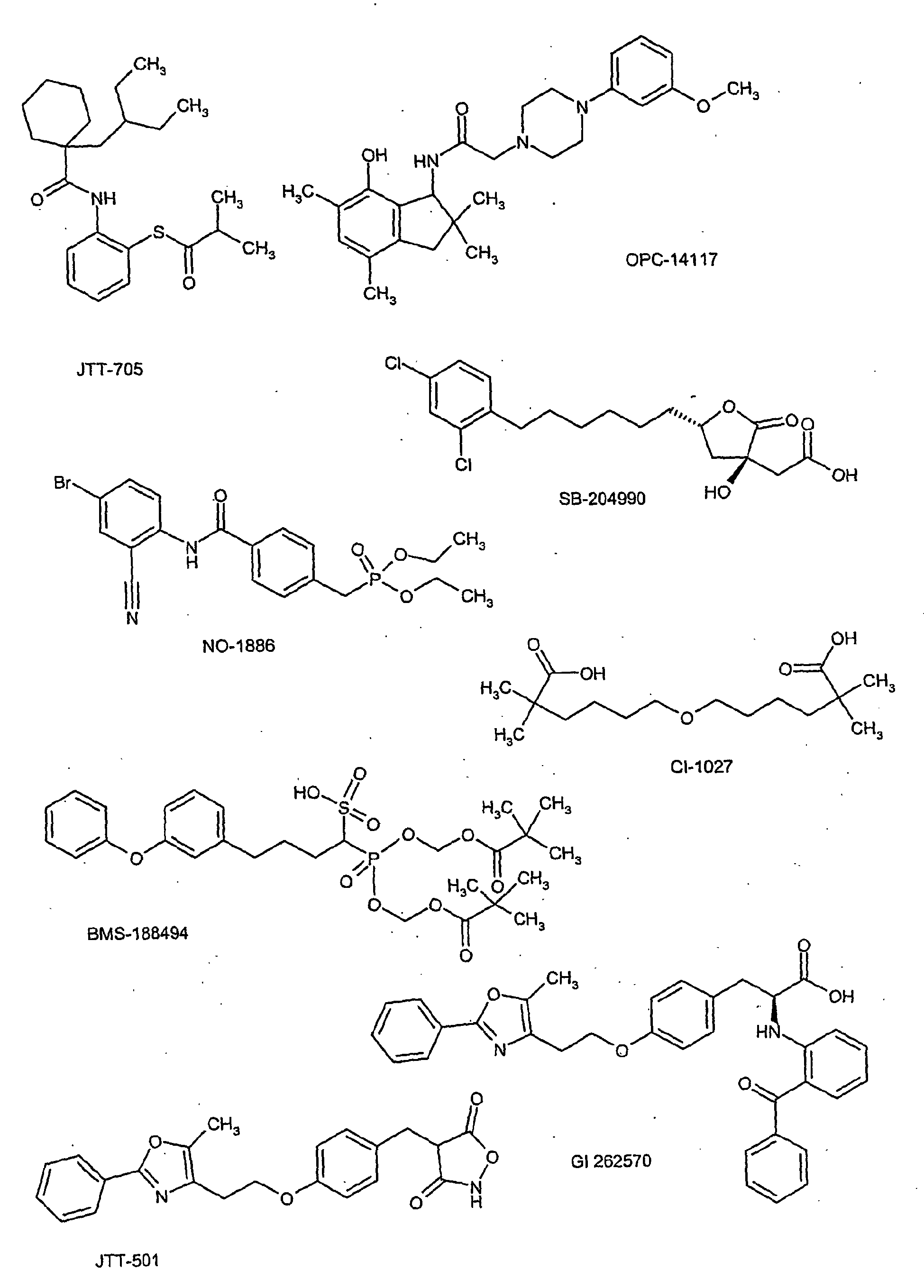

Die nachfolgend aufgeführten Beispiele dienen zur Erläuterung der Erfindung, ohne diese jedoch einzuschränken.

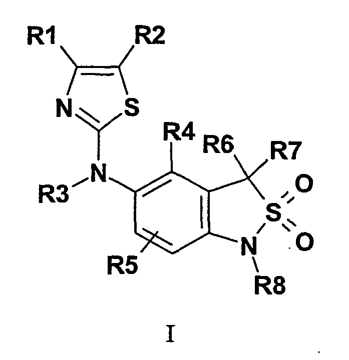

| **Bsp.** | **R1** | **R2** | **R3** | **R4** | **R5** | **R6** | **R7** | **R8** | **Fp.** |
|---|---|---|---|---|---|---|---|---|---|
| 1 | 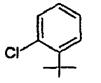 | H | H | H | H | H | H | H | |
| 2 | 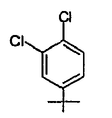 | H | H | H | H | H | H | H | |
| 3 | 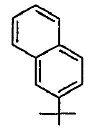 | H | H | H | H | H | H | H | |
| 4 | 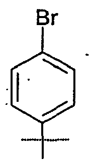 | H | H | H | H | H | H | H | |
| 5 | 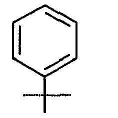 | CH₃ | H | H | h | h | h | h | |
| 6 | 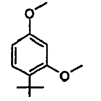 | H | H | H | H | H | H | H | |
| 7 | 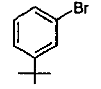 | H | H | H | H | H | H | H | |
| 8 | 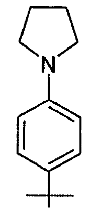 | H | H | H | H | H | H | H | |
| 9 | 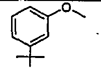 | H | H | H | H | H | H | H | |
| 10 | 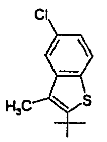 | H | H | H | H | H | H | H | |
| 11 | 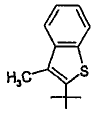 | H | H | H | H | H | H | H | |
| 12 | 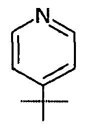 | H | H | H | H | H | H | H | |
| 13 | COOH | H | H | H | H | H | H | H | |
| 14 | 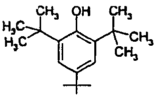 | H | H | H | H | H | H | H | |
| 15 | 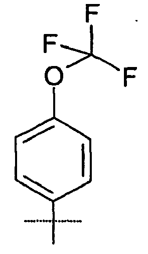 | H | H | H | H | H | H | H | |
| 16 | 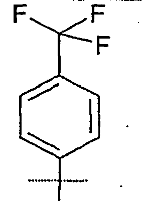 | H | H | H | H | H | H | H | |
| 17 | 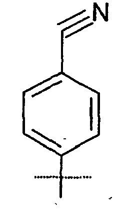 | H | H | H | H | H | H | H | |
| 18 | 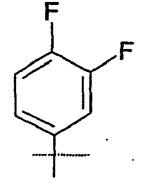 | H | H | H | H | H | H | H | |
| 19 | 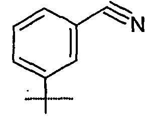 | H | H | H | H | H | H | H | |
| 20 | 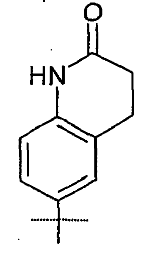 | H | H | H | H | H | H | H | |
| 21 | 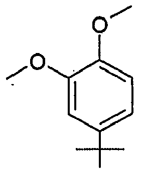 | H | H | H | H | H | H | H | |
| 22 | 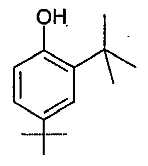 | H | H | H | H | H | H | H | |
| 23 | 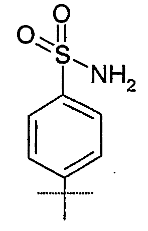 | H | H | H | H | H | H | H | |
| 24 | 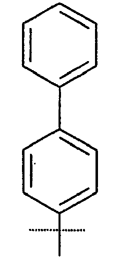 | H | H | H | H | H | H | H | |
| 25 | 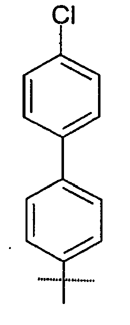 | H | H | H | H | H | H | H | |
| 26 | 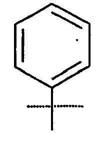 | 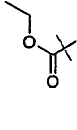 | H | H | H | H | H | H | |
| 27 | 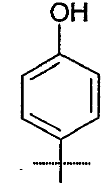 | H | H | H | H | H | H | H | |
| 28 | 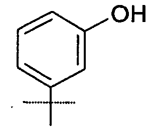 | H | H | H | H | H | H | H | |
| 29 | 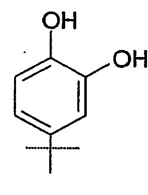 | H | H | H | H | H | H | H | |
| 30 | 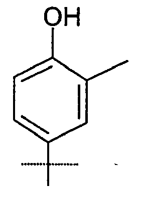 | H | H | H | H | H | H | H | |
| 31 | 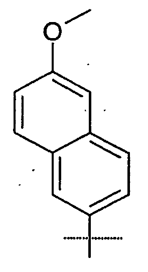 | H | H | H | H | H | H | H | |
| 32 | 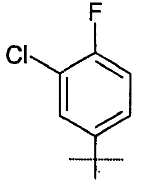 | H | H | H | H | H | H | H | |
| 33 | 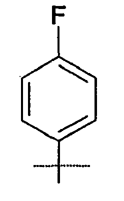 | 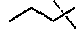 | H | H | H | H | H | H | |
| 34 | 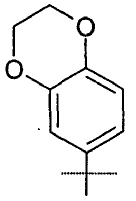 | H | H | H | H | H | H | H | |
| 35 | 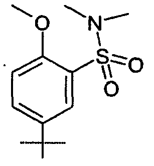 | H | H | H | H | H | H | H | |
| 36 | 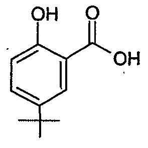 | H | H | H | H | H | H | H | |
| 37 | 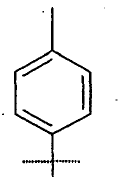 | 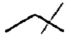 | H | H | H | H | H | H | |
| 38 | 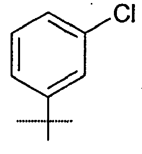 | CH₃ | H | H | H | H | H | H | |
| 39 | 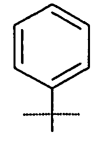 | 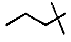 | H | H | H | H | H | H | |
| 40 | 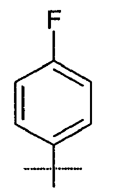 | CH₃ | H | H | H | H | H | H | |
| 41 | 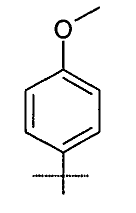 | CH₃ | H | H | H | H | H | H | |
| 42 | 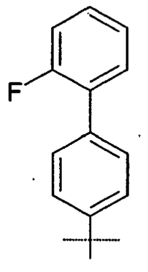 | CH₃ | H | H | H | H | H | H | |
| 43 | 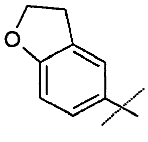 | H | H | H | H | H | H | H | |
| 44 | 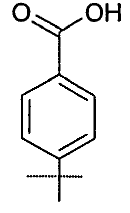 | H | H | H | H | H | H | H | |
| 45 | 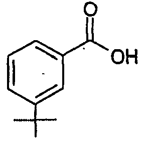 | H | H | H | H | H | H | H | |
| 46 | 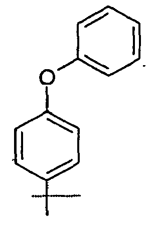 | H | H | H | H | H | H | H | |
| 47 | 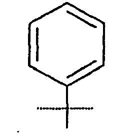 | H | H | H | H | H | H | H | |
| 48 | 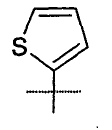 | H | H | H | H | H | H | H | |
| 49 | 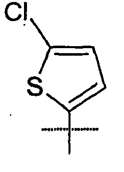 | H | H | H | H | H | H | H | |
| 50 | 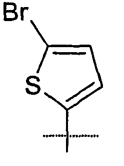 | H | H | H | H | H | H | H | |
| 51 | 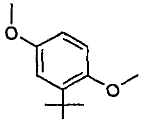 | H | H | H | H | H | H | H | |
| 52 | 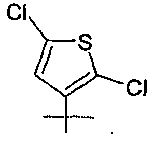 | H | H | H | H | H | H | H | |
| 53 | 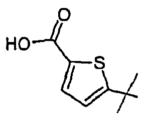 | H | H | H | H | H | H | H | |
| 54 | 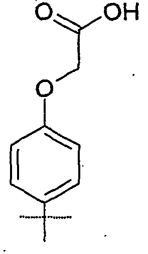 | H | H | H | H | H | H | H | |
| 55 | 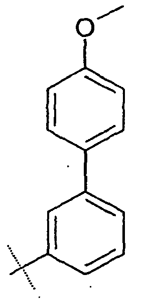 | H | H | H | H | H | H | H | |
| 56 | 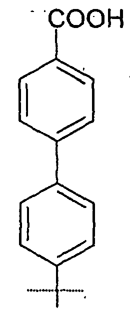 | H | H | H | H | H | H | H | |
| 57 | 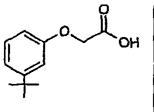 | H | H | H | H | H | H | H | |
| 58 | 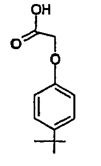 | H | 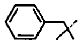 | H | H | H | H | H | |
| 64 | 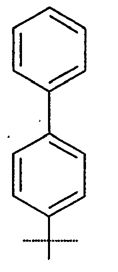 | H | 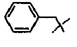 | H | H | H | H | H | |
| 65 | 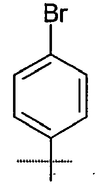 | H | 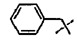 | H | H | H | H | H | |
| 66 | 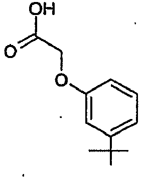 | H | 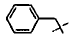 | H | H | H | H | H | |
| 67 | 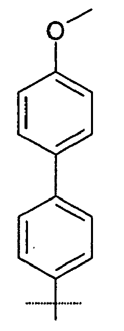 | H | H | H | H | H | H | H | |
| 68 | 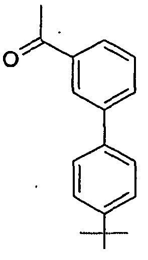 | H | H | H | H | H | H | H | |
| 69 | 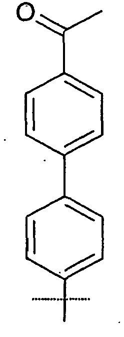 | H | H | H | H | H | H | H | |
| 72 | 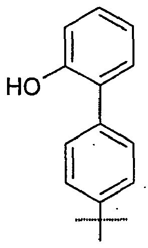 | H | H | H | H | H | H | H | H |
| 73 | 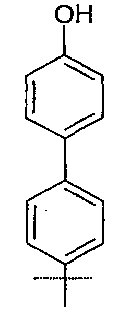 | H | H | H | H | H | H | H | H |
| 74 | 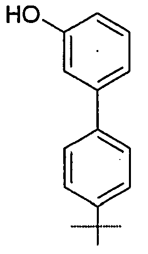 | H | H | H | H | H | H | H | |
| 75 | 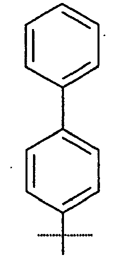 | 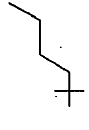 | H | H | H | H | H | H | |
| 76 | 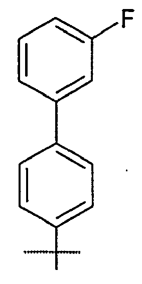 | H | H | H | H | H | H | H | |
| 77 | 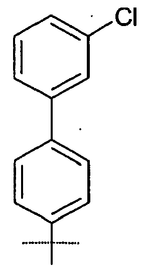 | H | H | H | H | H | H | H | |
| 78 | 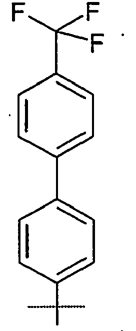 | H | H | H | H | H | H | H | |
| 79 | 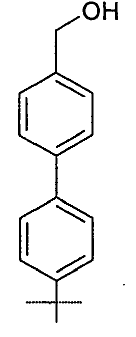 | H | H | H | H | H | H | H | |
| 80 | 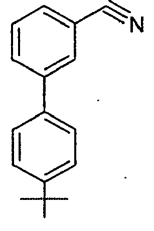 | H | H | H | H | H | H | H | |
| 81 | 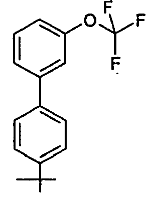 | H | H | H | H | H | H | H | |
| 82 | 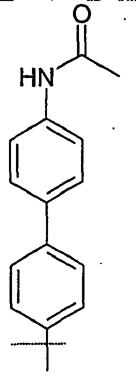 | H | H | H | H | H | H | H | |
| 83 | 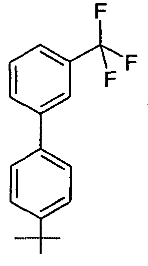 | H | H | H | H | H | H | H | |
| 84 | 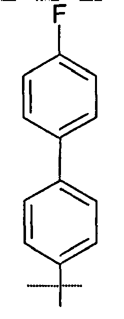 | H | H | H | H | H | H | H | |
| 85 | 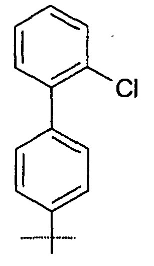 | H | H | H | H | H | H | H | |
| 86 | 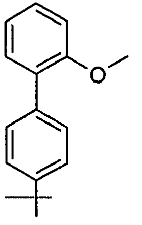 | H | H | H | H | H | H | H | |
| 87 | 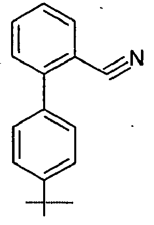 | H | H | H | H | H | H | H | |
| 88 | 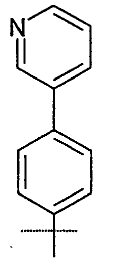 | H | H | H | H | H | H | H | |
| 89 | 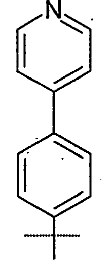 | H | H | H | H | H | H | H | |
| 90 | 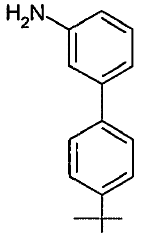 | H | H | H | H | H | H | H | |
| 91 | 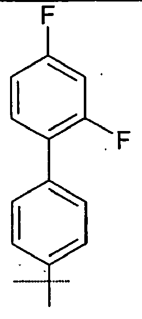 | H | H | H | H | H | H | H | |
| 92 | 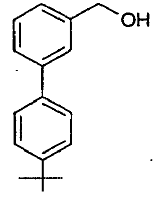 | H | H | H | H | H | H | H | |
| 93 | 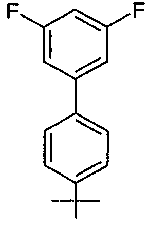 | H | H | H | H | H | H | H | |
| 94 | 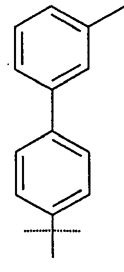 | H | H | H | H | H | H | H | |
| 95 | 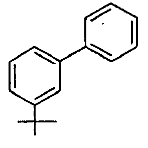 | H | H | H | H | H | H | H | |
| 96 | 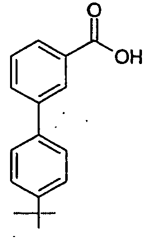 | H | H | H | H | H | H | H | |
| 106 | 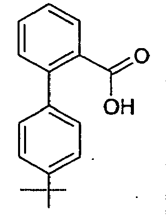 | H | H | H | H | H | H | H | |
| 107 | 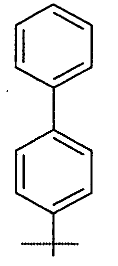 | H | 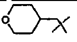 | H | H | H | H | H | |
| 108 | 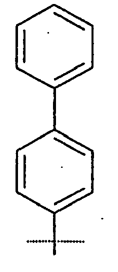 | 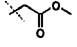 | 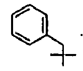 | H | H | H | H | H | |
| 109 | 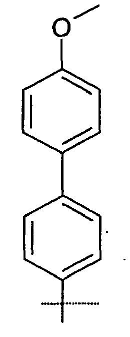 | 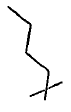 | 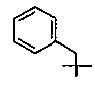 | H | H | H | H | H | |
| 110 | 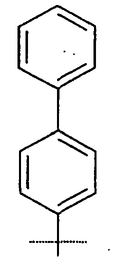 | CH₃ | 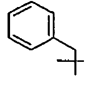 | H | H | H | H | H | |
| 111 | 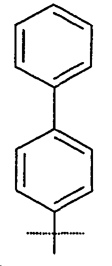 | 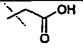 | 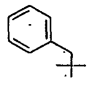 | H | H | H | H | H | |
| 114 | 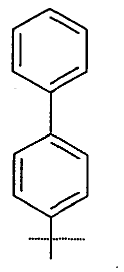 | H | 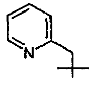 | H | H | H | H | H | |
| 115 | 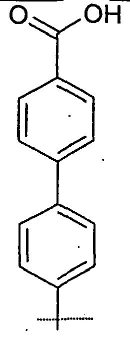 | H | 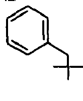 | H | H | H | H | H | |
| 116 | 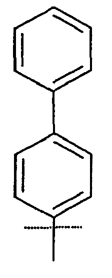 | H | 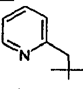 | H | H | H | H | H | |
| 117 | 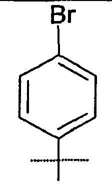 | H | 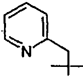 | H | H | H | H | H | |
| 118 | 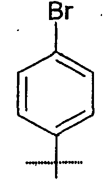 | H | 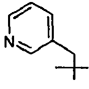 | H | H | H | H | H | |
| 119 | 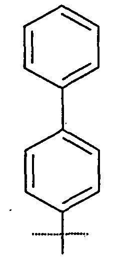 | H | 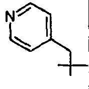 | H | H | H | H | H | |
| 120 | 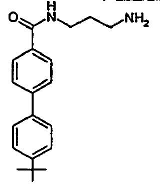 | H | H | H | H | H | H | H | |
| 121 | 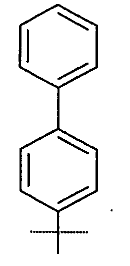 | H | 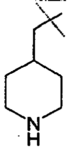 | H | H | H | H | H | |
| 122 | 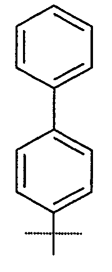 | H | 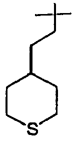 | H | H | H | H | H | |
| 123 | 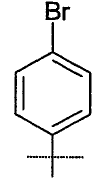 | H | 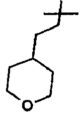 | H | H | H | H | H | |
| 124 | 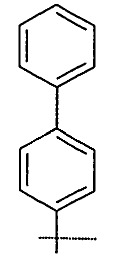 | H | 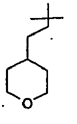 | H | H | H | H | H | |
| 125 | 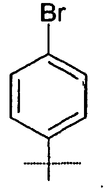 | 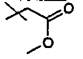 | H | H | H | H | H | H | |
| 126 | 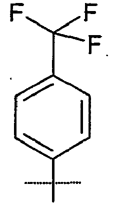 | 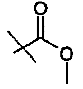 | H | H | H | H | H | H | |
| 127 | 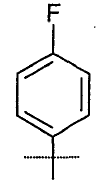 | 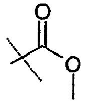 | H | H | H | H | H | H | |
| 128 | 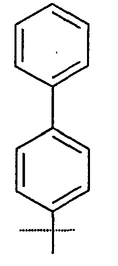 | H | 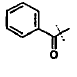 | H | H | H | H | H | |
| 129 | 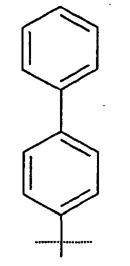 | H | 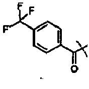 | H | H | H | H | H | |
| 130 | 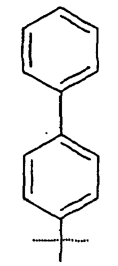 | H | 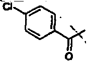 | H | H | H | H | H | |
| 131 | 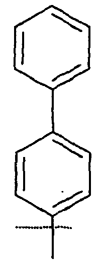 | H | 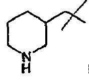 | H | H | H | H | H | |
| 132 | 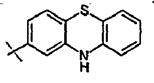 | H | H | H | H | H | H | H | |
| 133 | 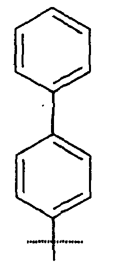 | H | 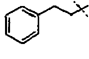 | H | H | H | H | H | |
| 134 | 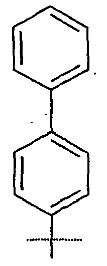 | H | 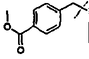 | H | H | H | H | H | |
| 135 | 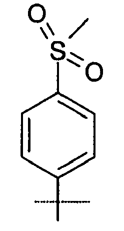 | H | H | H | H | H | H | H | |
| 138 | 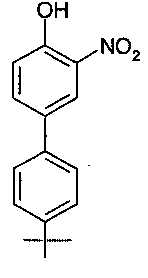 | H | H | H | H | H | H | H | |
| 139 | 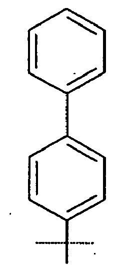 | H | 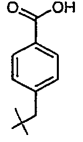 | H | H | H | H | H | |

Die Wirksamkeit der Verbindungen wurde wie folgt getestet:

Enzymatische Prüfsysteme zum Nachweis der Hemmung einer Phosphatase

Die Verbindungen der Formel I wurden in einem in vitro Assay auf ihre Phosphatase inhibierende Wirkung getestet. Die Enzympräparation und die Durchführung des Assays wurde wie folgt durchgeführt.

Gewinnung der Enzympräparation:
A) Zellkultur:
   Sf9 Zellen (=Zelltyp von Spodoptera frugiperda; erhältlich bei invitrogen) werden in Spinnerflaschen bei 28°C in Grace's supplementiertem Medium (Gibco-BRL) mit 10% Hitze-inaktiviertem fötalem Kälberserum (Gibco-BRL) gemäß dem Protokoll von Summers und Smith (A Manual for Methods for Baculovirus Vectors and Insect Culture Procedures [Bulletin No. 15555]. Texas A & M University, Texas Agricultural Experiment Station, College Station, TX, 1987) kultiviert.
   Konstruktion von rekombinanten Baculovirus Transfervektoren: cDNA kodierend für die regulatorischen and katalytischen Domänen der menschlichen PTP1B, aber ohne die carboxy-terminale hydrophobe Region (entsprechend 1-299 aa) wurde über Polymerasekettenreaktion über Primer mit angefügten Klonierungsstellen und geeigneten cDNA Matrizen (erhältlich beispielsweise von invitrogen) erhalten und dann in Baculovirusexpressionvektoren (Amersham Pharmacia Biotech.) kloniert. Die rekombinanten Baculoviren wurden mit Hilfe des Bac-to-Bac Baculovirus Expressionsystems (erhältlich von Gibco-BRL) hergestellt. Das Gen wurde in das pFASTBAC Donorplasmid kloniert (erhältlich von Life Technologies). Das resultierende Plasmid wurde in kompetente DH10BAC Escherichia coli Zellen (erhältlich von Life Technologies) transformiert. Nach der Transposition und Antibiotikaselektion wurde die rekombinante Plasmid-DNA von selektierten E. coli Kolonien isoliert und dann für die Transfektion von Sf9 Insektenzellen benutzt. Der Viruspartikel im Überstandsmedium wurde dreimal amplifiziert bis auf ein virales Stockvolumen von 500 ml.
B) Produktion von rekombinantem Protein:
   Baculovirusinfection einer 500-ml Spinnerkultur von Sf9 Zellen wurde im wesentlichen durchgeführt wie von Summers und Smith beschrieben (s.o.). Sf9 Zellen bei einer Dichte von 1-3 x 10⁶ Zellen/ml wurden durch Zentrifugation bei 300 g für 5 min pelletiert, der Überstand wurde entfernt und die Zellen in einer Dichte von 1 x 10⁷ Zellen/ml in einem geeigneten rekombinanten Viralstock (MOI 10) resuspendiert. Nach vorsichtigem Schütteln für 1.5 Std. bei Raumtemperatur wurde frisches Medium hinzugegeben, um eine Zelldichte von 1 x 10⁶ Zellen/ml zu erreichen. Die Zellen wurden dann in Suspension bei 28°C für geeignete Perioden nach Postinfektion kultiviert.
C) Zelluläre Fraktionierung und Gesamtzellextrakte von infizierten Sf9 Zellen:
   Nach der Postinfektion wurden Aliquots einer Analyse der Proteinexpression durch SDS-PAGE und Westernblotanalyse unterzogen. Die zelluläre Fraktionierung wurde durchgeführt wie beschrieben (Cromlish, W. and Kennedy, B. Biochem. Pharmacol. 52: 1777-1785, 1996). Gesamtzellextrakte wurden von 1-ml Aliquots der infizierten Sf9 Zellen nach bestimmten Zeiten Postinfektion gewonnen. Die pelletierten Zellen (300xg, 5 min) wurden einmal in Phosphate-gepufferter Saline (4°C) gewaschen, resuspendiert in 50 µl Wasser und durch wiederholtes Einfrieren/Auftauen aufgeschlossen.
      Proteinkonzentrationen wurden mit Hilfe der Bradfordmethode und Rinderserumalbumin als Standard bestimmt.

Durchführung des Assays:
A) Dephosphorylierung eines Phosphopeptids:
   Dieser Assay beruht auf der Freisetzung von Phosphat aus einem Konsensussubstratpeptid, welches im nanomolaren Konzentrationsbereich durch die Malachitgrün-Ammoniummolybdate-Methode (Lanzetta, P.A., Alvarez, L.J., Reinach, P.S., Candia, O.A. Anal Biochem. 100: 95-97, 1979) adaptiert für das Mikrotiterplattenformat nachgewiesen wird. Das Dodecatrisphosphopeptid, TRDIYETDYYRK (Biotrend, Köln) entpricht den Aminosäuren 1142-1153 der katalytischen Domaäne des Insulinrezeptors und wird (auto)phosphoryliert an den Tyrosinresten 1146, 1150, und 1151. Die rekombinante hPTP1B wurde mit Assaypuffer verdünnt (40 mM Tris/HCl, pH 7.4, 1 mM EDTA, 20 mM DTT), entsprechend einer Aktivität von 1000-1500 nmol/min/mg Protein und (eine 20 µl-Portion) dann vorinkubiert (15 min, 30°C) in Ab- oder Anwesenheit der Testsubstanz (5 µl) in der gewünschten Konzentration (Endkonz. DMSO 2 % max.) in einem Gesamtvolumen von 90 µl (Assaypuffer). Zum Start der Dephosphorylierungsreaktion wurde das Peptidsubstrat (10 µl, vorgewärmt auf 30°C) zur vorinkubierten Enzympräparation mit oder ohne Testsubstanz (Endkonz. 0.2-200 µM) hinzugegeben und die Inkubation für 1 Std. fortgesetzt. Die Reaktion wurde beendet durch Hinzufügen von 100 µl Malachitgrünhydrochlorid (0.45 %, 3 Teile), Ammoniummolybdattetrahydrat (4.2 % in 4 N HCl, 1 Teil) und 0.5 % Tween 20 als Stoplösung. Nach 30 min Inkubation bei 22°C für die Entwicklung der Farbe wurde die Absorption bei 650 nm mit Hilfe eines Mikrotiterplattenlesegeräts (Molecular Devices) bestimmt. Proben und Leerwerte wurden als Dreifachwerte gemessen. Die PTP1B Aktivität wurde als Nanomole an freigesetztem Phosphat pro min und mg Protein mit Kaliumphosphat als Standard berechnet. DIe Inhibition der rekombinanten hPTP1B durch Testsubstanzen wurde als Prozent der Phosphatasekontrolle berechnet. Die IC₅₀-Werte zeigen signifikante Übereinstimmung mit einer Vier-Parameter-nichtlinearen logistischen Regressionskurve.
B) Spaltung von p-Nitrophenylphosphat:
   Dieser Assay beruht auf der Absorptionsveränderung des nicht-physiologischen Substrats p-Nitrophenylphosphat während der Spaltung zu Nitrophenol unter Standardbedingungen (Tonks, N.K., Diltz, C.D:, Fischer, E.H. J. Biol. Chem. 263: 6731-6737, 1988; Burke T.R., Ye, B., Yan, X.J., Wang, S.M., Jia, Z.C., Chen, L., Zhang, Z.Y., Barford, D. Biochemistry 35: 15989-15996, 1996). Die Inhibitoren werden in geeigneter Verdünnung zu den Reaktionsgemischen pipettiert, die 0.5-5 mM p-Nitrophenylphosphat enthalten. Die folgenden Puffer wurden benutzt (Gesamtvolumen 100 µl): (a) 100 mM Natriumazetat (pH 5.5), 50 mM NaCl, 0.1 % (w/v) Rinderserumalbumin, 5 mM Glutathion, 5 mM DTT, 0.4 mM EGTA und 1 mM EDTA; (b) 50 mM Hepes/KOH (pH 7.4), 100 mM NaCl, 0.1 % (w/v) Rinderserumalbumin, 5 mM Glutathion, 5 mM DTT und 1 mM EDTA. Die Reaktion wurde gestartet durch Zugabe von Enzym und in Mikrotiterplatten bei 25°C für 1 Std. durchgeführt. Die Reaktion wurde beendet durch Zugabe 100 µl 0.2 N NaOH. Die Enzymaktivität wurde bestimmt durch Messung der Absorption bei 405 nm mit geeigneten Korrekturen für Absorption der Testsubstanzen und von p-Nitrophenylphosphat. Die Ergebnisse wurden als Prozent der Kontrolle ausgedrückt, in dem die Menge an gebildetem p-Nitrophenol in den Testsubstanz-behandelten Proben (nmol/min/mg Protein) mit der Menge in den unbehandelten Proben verglichen wurde. Der Mittelwert und die Standardabweichung wurden berechnet, die IC50-Werte wurden durch Regressionsanalyse des linearen Anteils der Hemmkurven bestimmt.

**Tabelle 2: Biologische Aktivität**

| **Bsp.** | **IC-50 (µM)** |
|---|---|
| 1 | >80 |
| 2 | 27,8 |
| 3 | 8,8 |
| 4 | 6,2 |
| 5 | 15,9 |
| 6 | 48,0 |
| 7 | 7,9 |
| 8 | 5,9 |
| 9 | 6,3 |
| 10 | 5,3 |
| 11 | 6,2 |
| 12 | >80 |
| 13 | >80 |
| 14 | 2,77 |

Aus der Tabelle ist abzulesen, dass die Verbindungen der Formel I die Aktivität der Phosphotyrosinphosphatase 1B (PTP1B) hemmen und dadurch zur Senkung des Blutzuckerspiegels gut geeignet sind. Sie eignen sich damit insbesonders zur Behandlung von Diabetes Typ I und II, von Insulinresistenz, von Dyslipidämien, des metabolischen Syndroms / Syndrom X, von krankhafter Fettleibigkeit und zur Gewichtsreduktion bei Säugetieren. Weiterhin eignen sich Verbindungen der Formel I, wegen ihrer Hemmung der PTP1B, zur Behandlung von Hyperglyzeridämie, Bluthochdruck, Atherosklerose, Fehlfunktionen des Immunsystems, Autoimmunkrankheiten, allergischen Krankheiten wie z.B. Asthma, Arthritis, Osteoarthritis, Osteoporose, Proliferationsstörungen wie Krebs und Psoriasis, Krankheiten mit verminderter oder erhöhter Produktion von Wachstumsfaktoren, Hormonen oder Cytokinen, die die Freisetzung von Wachstumshormonen auslösen.
Die Verbindungen eignen sich auch zur Behandlung Erkrankungen des Nervensystems, wie zum Beispiel Alzheimer oder Multiple Sklerose.
Die Verbindungen eignen sich auch zur Behandlung von Störungen des Empfindens und anderer psychiatrischen Indikationen, wie zum Beispiel Depressionen, Angstzuständen, Angstneurosen, Schizophrenie, zur Behandlung von Störungen assoziiert mit dem zirkadianen Rhythmus und zur Behandlung von Drogenmissbrauch.
Weiterhin eignen sie sich zur Behandlung von Schlafstörungen, Schlaf Apnoe, weiblicher und männlicher Sexualstörungen, Entzündungen, Akne, Pigmentierung der Haut, Störungen des Steroidstoffwechsels, Hautkrankheiten und Mykosen.

Nachfolgend wird die Herstellung einiger Beispiele detailliert beschrieben, die übrigen Verbindungen der Formel I wurden analog erhalten:

Experimenteller Teil:

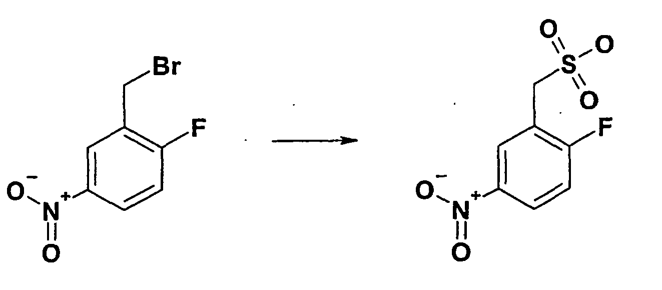

Zu einer Lösung von Na₂SO₃ (27,36 g, 0,128 mol) in H₂O (375 mL) wird eine Lösung von (2-Fluor-5-nitrobenzylbromid (30 g, 0,128 mol) in Acetonitril (250 mL) zugegeben und der Ansatz 24 h bei RT gerührt. Das Lösungsmittel wird im Vakuum abdestilliert, der Rückstand mit 100 ml Isopropanaol verrührt, der Feststoff abfiltriert und mit wenig Isopropanol bzw. Diethylether gewaschen.
Ausbeute: 28,15 g

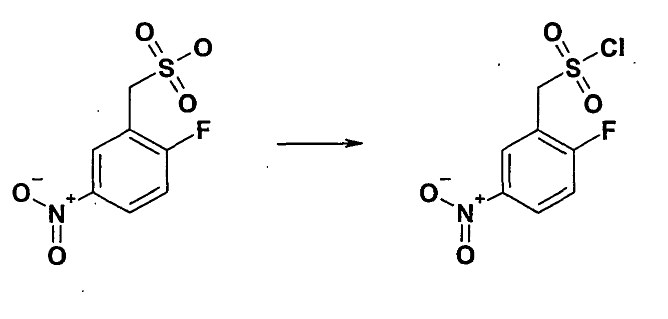

Das Natriumsalz der Sulfonsäure 1 (35,19 g, 0,1368 mol) wird in POCl₃ (430 mL) vorgelegt und anschließend PCl₅ (28,78 g, 0,137 mol) zugegeben. Der Ansatz wird 5 h unter Rückfluß erhitzt. Zur Aufarbeitung wird im Vakuum eingeengt, und der Rückstand auf Eis/Wasser gegossen. Das Reaktionsprodukt scheidet sich dabei als hellgelber Feststoff ab, welcher abfiltriert wird
Ausbeute: 30,3 g

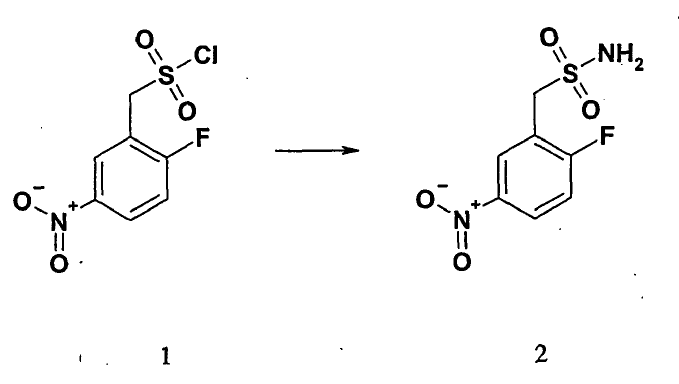

Eine Lösung des Sulfonsäurechlorids 1 (30,3 g, 0,12 mol) in CH₂Cl₂ (125 mL) wird zu konz Ammoniak (90 mL, 1,2 mol) bei RT zugetropft. Der Ansatz wird 20 h bei RT gerührt, und anschließend mit HCl (1n) auf pH 1 angesäuert. Anschließend wird die organische Phase unter reduziertem Druck abdestilliert, wobei das Reaktionsprodukt als hellgelber Feststoff abscheidet. Das Reaktionsprodukt wird anschließend abfiltriert.
Ausbeute 25,01 g (89,4%).

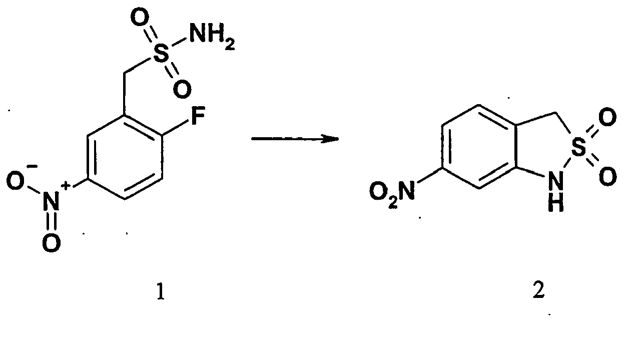

Zu einer Lösung von Verbindung 1 (25 g, 0,107 mol) in DMF (1 L) wird bei RT Diazabicycloundecen (34,1 g, 33,42 mL, 0,22 mol) zugegeben und das Reaktionsgemisch 2 h bei 130° C gerührt. Anschließend wir das Lösungsmittel im Vakuum abdestilliert, der Rückstand mit Wasser (400 mL) versetzt, mit HCl (2n, 400 mL) versetzt und das Produkt mehrfach mit Dichlormethan extrahiert. Die vereinigten organischen Phasen werden getrocknet (Na₂SO₄) und das Lösungsmittel unter reduziertem Druck abdestilliert. Der verbleibende Rückstand wird mit wenig kaltem Isopropanol verrührt und das Reaktionsprodukt anschließend abfiltriert.
Ausbeute: 20,8 g (91,3 %).

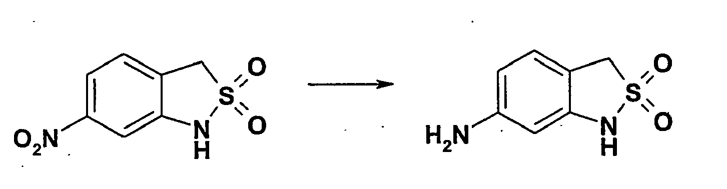

535 mg der Nitroverbindung werden in 100 mL Methanol/THF-Gemisch (1:1) gelöst und mit 5 mol % Pd (10% auf Aktivkohle) versetzt. Anschließend wird mit Wasserstoff in einer Hydrierapparatur bei Zimmertemperatur bis zur Beendigung der Wasserstoffaufnahme hydriert (Reaktionszeit: 1 h).
Zur Aufarbeitung wird der Katalysator über Celite® Filterhilfe abfiltriert und das Filtrat unter reduziertem Druck eingeengt. Der ölige Rückstand wird mit wenig Diethylether verrührt, abfiltriert, mit n-Pentan gewaschen und im Vakuumgetrocknet.
Ausbeute: 397 mg (86 % d. Th.) 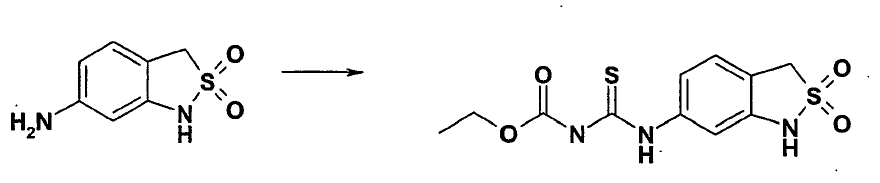 368 mg (2 mmol) des oben hergestellten Amins werden in 30 ml abs. THF gelöst und unter Rühren bei Zimmertemperatur mit 250 µl Ethoxycarbonylisothiocyanat versetzt. Anschließend wird 4 h bei Zimmertemperatur gerührt.

Zur Aufarbeitung wird das Solvens unter reduziertem Druck entfernt, der ölige Rückstand mit Diethylether verrührt und das Reaktionsprodukt abfiltriert.
Ausbeute 613 mg (97% d. Th.), beige Kristalle 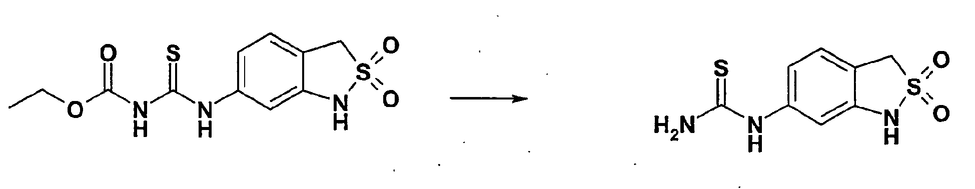 504 mg (1,6 mmol) des Carbamats werden in 10 mL THF/Wasser (1:1) suspendiert und unter Rühren bei Zimmertemperatur mit 3.2 ml 3.2 (mmol) 1M wässr. NaOH versetzt. Das Reaktionsgemisch wird 5 Stunden bei Zimmertemperatur gerührt.

Zur Aufarbeitung wird unter reduziertem Druck auf ca. 1/3 des ursprünglichen Volumens eingeengt und das Reaktionsgemisch mittels Zugabe von wässriger 2n HCI-Lösung auf pH 6 eingestellt, wobei der erhaltene Thioharnstoff langsam in Form hellbeiger Kristalle ausfällt. Das Reaktionsprodukt wird abfiltriert und mit Wasser gewaschen.
Ausbeute 300 mg (77 % d. Th.)

### Beispiel 1:

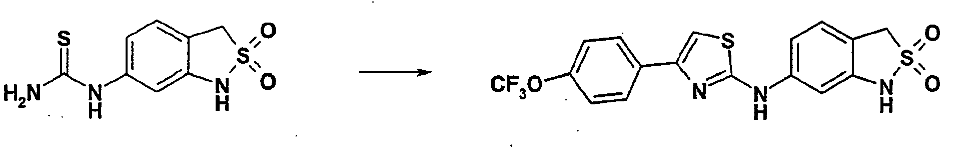

73 mg (0.3 mmol) des Thioharnstoffs werden in 5 ml abs. Ethanol gelöst und mit 85 mg (0.3 mmol) 4-(Trifluormethoxy)phenacylbromid versetzt. Das Reaktionsgemisch wird 5 Stunden unter Rückfluss des Lösungsmittels
Zur Aufarbeitung wird das Lösungsmittel unter reduziertem Druck entfernt und der Rückstand mittels Chromatographie an Kieselgel (15 - 25 µ; Fa: Merck) mit Ethylacetat/n-Heptan,
Mischungsverhältnis 1:1, als mobiler Phase gereinigt.
Ausbeute 79 mg (61 % d. Th.)

## Patentansprüche

1. Verbindungen der Formel I, 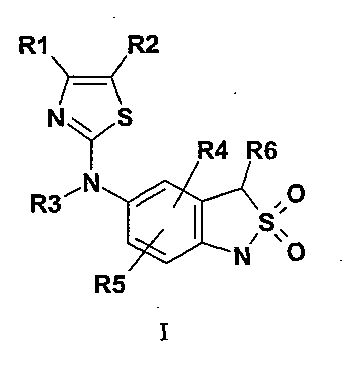 worin bedeuten
R1, R2 unabhängig voneinander H, Aryl, COOH, (C₁-C₆)-Alkylen-COOH, -COO(C₁₋C₆)-Alkyl, (C₁-C₆)-Alkylen-COO(C₁-C₆)-Alkyl, (C₁-C₆)-Alkyl, (C₂-C₆)-Alkenyl, (C₁-C₆)-alkylen-Aryl, Heterocyclus, (C₁-C₆)-alkylen-Heterocyclus, CF₃, OCF₃, CN, (CH₂)₁₋₆-OH, O-(C₁-C₆)-Alkyl, CO-(C₁-C₆)-Alkyl, -C(O)O-Alkyl, COOH, CON(R9)(R10) wobei die Arylreste sowie die heterocyclischen Reste ein oder mehrfach substituiert sein können mit F, Cl, Br, (CH₂)₀₋₂OH, (C₁-C₆)-Alkyl, (C₂₋C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆-Alkylen)-Piperazinon, Morpholin, Thiomorpholin, NO₂, CN, O-(C₁-C₆)-Alkyl, S(O)₀₋₂-(C₁-C₆)-Alkyl, SO₂-N(R9)(R10), CO-(C₁-C₆)-Alkyl, -COOH, (C₁-C₆)-Alkylen-COOH, COO(C₁₋C₆)-Alkyl, (C₁-C₆)-Alkylen-COO(C₁-C₆)-Alkyl, (C₃-C₁₀)-Cycloalkyl, Phenyl, wobei diese Piperidinon, Piperazin, Piperazinon, N-(C₁-C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁-C₆)-Alkyl, CF₃, OCF₃, N(R9)(R10);
R3 H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl, wobei die Aryl- und Heterocyclischen Reste ein oder mehrfach mit F, Cl, Br, (C₁-C₆)-Alkyl, COOH, COO(C₁-C₆)-Alkyl, CF₃ oder OCF₃ substituiert sein können;
R4, R5 unabhängig voneinander H, F, Cl, Br, (C₁-C₆)-Alkyl, CF₃, OCF₃, NO₂, N(R9)(R10), CN, O-(C₁-C₆)-Alkyl, CO-(C₁-C₆)-Alkyl, COOH, (C₁-C₆)-Alkylen-COOH, CON(R9)(R10), (C₁-C₆)-Alkylen-CON(R9)(R10), COO(C₁-C₆)-Alkyl, (C₁-C₆)-Alkylen-COO(C₁-C₆)-Alkyl, S(O)₀₋₂-(C₁-C₆)-Alkyl, S(O)₂-N(R9)(R10), CH₂OH, CH₂OCH₃;
R6, R7 unabhängig voneinander H, F, Cl, Br, (C₁-C₆)-Alkyl, Cyclopropyl, Tetrafluorocyclopropyl, Difluorcyclopropyl; oder
R6 und R7 bilden gemeinsam die Gruppe =CH₂;
R 8 H, CH₃, CF₃, CH₂OH;
R 9 H, (C₁-C₄)-Alkyl;
R10 H, (C₁-C₄)-Alkyl; oder
R 9 und R10 bilden gemeinsam mit dem N-Atom, an das sie gebunden sind, ein 3-9 gliedriges Ringsystem;
sowie deren physiologisch verträgliche Salze.

2. Verbindungen der Formel I, gemäß Anspruch 1, **dadurch gekennzeichnet, daß** darin bedeuten
R1 Aryl, (C₁-C₆)-Alkyl, (C₂-C₆)-Alkenyl, (C₁-C₆)-alkylen-Aryl, Heterocyclus, (C₁₋C₆)-alkylen-Heterocyclus, CF₃, OCF₃, CN, (CH₂)₁₋₆-OH, O-(C₁-C₆)-Alkyl, CO-(C₁-C₆)-Alkyl, C(O)O-alkyl, COOH, CON(R9)(R10), wobei die Arylreste sowie die heterocyclischen Reste ein oder mehrfach substituiert sein können mit F, Cl, Br, (CH₂)₀₋₂OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆-Alkylen)-Piperazinon, Morpholin, Thiomorpholin, NO₂, CN, O-(C₁₋C₆)-Alkyl, S(O)₀₋₂-(C₁-C₆)-Alkyl, SO₂-N(R9)(R10), CO-(C₁-C₆)-Alkyl, -COOH, (C₁-C₆)-Alkylen-COOH, -COO(C₁-C₆)-Alkyl, (C₀-C₆)-Alkylen -COO(C₁-C₆)-Alkyl, C₃-C₁₀-Cycloalkyl, Phenyl, wobei diese Piperidinon, Piperazin, Piperazinon, N-(C₁-C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁-C₆)-Alkyl, CF₃, OCF₃, N(R9)(R10);
R2 H, Aryl, COOH, (C₁-C₆)-Alkylen-COOH, -COO(C₁-C₆)-Alkyl, (C₁-C₆)-Alkylen-COO(C₁-C₆)-Alkyl; (C₁-C₆)-Alkyl, (C₂-C₆)-Alkenyl, (C₁-C₆)-alkylen-Aryl, Heterocyclus, (C₁-C₆)-alkylen-Heterocyclus, CF₃, OCF₃, CN, -(CH₂)₁₋₆-OH, O-(C₁-C₆)-Alkyl, CO-(C₁-C₆)-Akyl, C(O)O-alkyl, COOH, CON(R9)(R10), wobei die Arylreste sowie die heterocyclischen Reste ein oder mehrfach substituiert sein können mit F, Cl, Br, (CH₂)₀₋₂OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆₋Alkylen)-Piperazin, N-(C₁-C₆-Alkylen)-Piperazinon, Morpholin, Thiomorpholin, NO₂, CN, O-(C₁-C₆)-Alkyl,, S(O)₀₋₂-(C₁-C₆)-Alkyl, SO₂-N(R9)(R10), CO-(C₁₋C₆)-Alkyl, -COOH, (C₁-C₆)-Alkylen-COOH, -COO(C₁-C₆)-Alkyl, (C₁-C₆)-Alkylen-COO(C₁-C₆)-Alkyl, C₃-C₁₀-Cycloalkyl, Phenyl;
R3 H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl;
R4, R5 unabhängig voneinander H, F, Cl, Br, (C₁-C₆)-Alkyl, CF₃, OCF₃, NO₂, N(R9)(R10), CN, O-(C₁-C₆)-Alkyl, CO-(C₁-C₆)-Alkyl, COOH, (C₁-C₆)-Alkylen-COOH, -CON(R9)(R10), (C₁-C₆)-Alkylen-CON(R9)(R10), COO(C₁-C₆)-Alkyl, (C₁C₆)-Alkylen-COO(C₁-C₆)-Alkyl, S(O)₀₋₂-(C₁-C₆)-Alkyl, S(O)₂-N(R9)(R10), CH₂OH, CH₂OCH₃;
R6, R7 unabhängig voneinander H, F, Cl, Br, (C₁-C₆)-Alkyl, Cyclopropyl, Tetrafluorocyclopropyl, Difluorcyclopropyl; oder
R6 und R7 bilden gemeinsam die Gruppe =CH₂;
R8 H, CH₃, CF₃, CH₂OH;
R9 H, (C₁-C₄)-Alkyl;
R10 H, (C₁-C₄)-Alkyl; oder
R9 und R10 bilden gemeinsam mit dem N-Atom, an das sie gebunden sind, ein 3-9 gliedriges Ringsystem;
sowie deren physiologisch verträgliche Salze.

3. Verbindungen der Formel I, gemäß Anspruch 1 oder 2, **dadurch gekennzeichnet, daß** darin bedeuten
R1 Phenyl, Naphthyl, Thionaphthyl, Pyridyl, wobei Phenyl, Naphthyl, Thionaphthyl und Pyridyl ein oder mehrfach substituiert sein können mit F, Cl, Br, (CH₂)₀₋₂ OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆₋Alkylen)-Piperazinon, Morpholin; Thiomorpholin, NO₂, CN, O-(C₁-C₆)-Alkyl, S(O)₀₋₂-(C₁-C₆)-Alkyl, SO₂-N(R9)(R10), CO-(C₁-C₆)-Alkyl, COOH, (C₁-C₆)-Alkylen-COOH, COO(C₁-C₆)-Alkyl, (C₁-C₆)-Alkylen-COO(C₁-C₆)-Alkyl, C₃₋C₁₀-Cycloalkyl, Phenyl, wobei diese Piperidinon, Piperazin, Piperazinon, N-(C₁₋C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁₋C₆)-Alkyl, CF₃, OCF₃, N(R9)(R10);
R2 H, (C₁-C₆)-Alkyl, COOH, (C₁-C₆)-Alkylen-COOH, -COO(C₁-C₆)-Alkyl, (C₁₋C₆)-Alkylen-COO(C₁-C₆)-Alkyl;
R3 H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl;
R4, R5 H;
R6, R7 H;
R8 H;
R9 H, (C₁-C₄)-Alkyl;
R10 H, (C₁-C₄)-Alkyl;
sowie deren physiologisch verträgliche Salze.

4. Verbindungen der Formel I, gemäß einem oder mehreren der Ansprüche 1 bis 3, **dadurch gekennzeichnet, daß** darin bedeuten
R1 Phenyl, wobei Phenyl ein oder mehrfach substituiert sein kann mit F, Cl, Br, (CH₂)₀₋₂OH, (C₁-C₆)-Alkyl, (C₂-C₆)-Alkenyl, (C₂-C₆)-Alkinyl, CF₃, OCF₃, N(R9)(R10), Piperidinon, Piperazin, Piperazinon, N-(C₁-C₆-Alkylen)-Piperazin, N-(C₁-C₆-Alkylen)-Piperazinon, Morpholin, Thiomorpholin, NO₂, CN, O-(C₁₋C₆)-Alkyl, S(O)₀₋₂-(C₁-C₆)-Alkyl, SO₂-N(R9)(R10), CO-(C₁-C₆)-Alkyl, COOH, (C₁-C₆)-Alkylen-COOH, COO(C₁-C₆)-Alkyl, (C₁-C₆)-Alkylen-COO(C₁-C₆)-Alkyl, C₃-C₁₀-Cycloalkyl, Phenyl, wobei diese Piperidinon, Piperazin, Piperazinon, N-(C₁-C₆-Alkylen)-Piperazin, N-(C₁-C₆-Alkylen)-Piperazinon, Morpholin, Thiomorpholin und Phenylringe ein oder mehrfach substituiert sein können mit F, Cl, Br, (CH₂)₀₋₂-OH, COOH, CN, NO₂, -O-(C₁-C₆)-Alkyl, -NH-O-(C₁-C₆)-Alkyl, -(CO)-NH-O-(C₁-C₆)-Alkylen-N(R9)(R10), -(CO)-(C₁-C₆)-Alkyl, -(C₁-C₆)-Alkyl, CF₃, OCF₃, N(R9)(R10);
R2 H, (C₁-C₆)-Alkyl, -C(O)O-(C₁-C₆)-Alkyl, -(C₁-C₆)-Alkylen-C(O)O-(C₁-C₆)-Alkyl, -COOH, -(C₁-C₆)-Alkylen-COOH;
R3 H, (C₁-C₆)-Alkyl, (C₁-C₆)-Alkylen-Aryl, -C(O)-Aryl, (C₁-C₆)-Alkylen-Heterocyclus, CO-(C₁-C₆)-Alkyl;
R4, R5 H;
R6, R7 H;
R8 H;
R9 H;
R10 H
sowie deren physiologisch verträgliche Salze.

5. Verwendung der Verbindungen gemäß einem oder mehreren der Ansprüche 1 bis 4 zur Herstellung eines Arzneimittels.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocytstimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verwendung der Verbindungen gemäß einem oder mehreren der Ansprüche 1 bis 4 zur Herstellung eines Medikamentes zur Blutzuckersenkung.

10. Verwendung der Verbindungen gemäß einem oder mehreren der Ansprüche 1 bis 4 zur Herstellung eines Medikamentes zur Behandlung des Typ II Diabetes.

11. Verwendung der Verbindungen gemäß einem oder mehreren der Ansprüche 1 bis 4 zur Herstellung eines Medikamentes zur Behandlung von Lipid- und Kohlenhydratstoffwechselstörungen.

12. Verwendung der Verbindungen gemäß einem oder mehreren der Ansprüche 1 bis 4 zur Herstellung eines Medikaments zur Behandlung arteriosklerotischer Erscheinungen.

13. Verwendung der Verbindungen gemäß einem oder mehreren der Ansprüche 1 bis 4 zur Herstellung eines Medikaments zur Behandlung von Insulin Resistenz.

14. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. A compound of the formula I 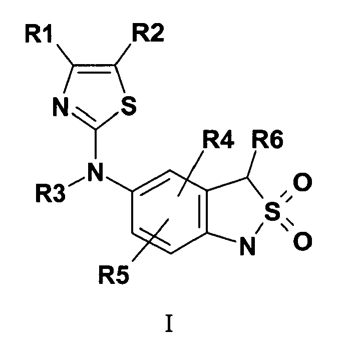 in which the meanings are
R1, R2 independently of one another H, aryl, COOH, (C₁-C₆)-alkylene-COOH, -COO(C₁-C₆)-alkyl, (C₁-C₆)-alkylene-COO(C₁C₆)-alkyl, (C₁-C₆)-alkyl, (C₂-C₆)-alkenyl, (C₁-C₆)-alkylene-aryl, heterocycle, (C₁-C₆)-alkylene-heterocycle, CF₃, OCF₃, CN, (CH₂)₁₋₆-OH, O-(C₁₋C₆)-alkyl, CO-(C₁-C₆)-alkyl, -C(O)O-alkyl, COOH, CON(R9)(R10), where the aryl radicals and the heterocyclic radicals may be substituted one or more times by F, Cl, Br, (CH₂)₀₋₂OH, (C₁-C₆)-alkyl, (C₂-C₆)-alkenyl, (C₂-C₆)-alkynyl, CF₃, OCF₃, N(R9)(R10), piperidinone, piperazine, piperazinone, N-(C₁-C₆₋alkylene)piperazine, N-(C₁-C₆-alkylene)piperazinone, morpholine, thiomorpholine, NO₂, CN, O-(C₁-C₆)-alkyl, S(O)₀₋₂-(C₁-C₆)-alkyl, SO₂-N(R9)(R10), CO-(C₁-C₆)-alkyl, -COOH, (C₁-C₆)-alkylene-COOH, COO(C₁-C₆)-alkyl, (C₁-C₆)-alkylene-COO(C₁-C₆)-alkyl, (C₃-C₁₀)-cycloalkyl, phenyl, where these piperidinone, piperazine, piperazinone, N-(C₁-C₆)-alkylene)piperazine, N-(C₁-C₆₋alkylene)piperazinone, morpholine, thiomorpholine and phenylrings may be substituted one or more times by F, Cl, Br, (CH₂)₀₋₂OH, COOH, CN, NO₂, -O-(C₁-C₆)-alkyl, -NH-O-(C₁-C₆)-alkyl, -(CO)-NH-O-(C₁-C₆)-alkylene-N(R9)(R10), -(CO)-(C₁-C₆)-alkyl, -(C₁-C₆)-alkyl, CF₃, OCF₃, N(R9)(R10);
R3 H, (C₁-C₆)-alkyl, (C₁-C₆)-alkylene-aryl, -C(O)-aryl, )C₁-C₆)-alkylene-heterocycle, CO-(C₁-C₆)-alkyl, where the aryl and heterocyclic radicals may be substituted one or more times by F, Cl, Br, (C₁-C₆)-alkyl, COOH, COO(C₁-C₆)-alkyl, CF₃ or OCF₃;
R4, R5 independently of one another H, F, Cl, Br, (C₁-C₆)-alkyl, CF₃, OCF₃, NO₂, N(R9)(R10), CN, O-(C₁-C₆)-alkyl, CO-(C₁-C₆)-alkyl, COOH, (C₁-C₆)-alkylene-COOH, CON(R9)(R10), (C₁-C₆)-alkylene-CON(R9)(R10), COO(C₁-C₆)-alkyl, (C₁-C₆)-alkylene-COO(C₁-C₆)-alkyl, S(O)₀₋₂-(C₁-C₆)-alkyl, S(O)₂-N(R9)(R10), CH₂OH, CH₂OCH₃;
R6, R7 independently of one another H, F, Cl, Br, (C₁-C₆)-alkyl, cyclopropyl, tetrafluorocyclopropyl, difluorocyclopropyl; or
R6 and R7 together form the group =CH₂;
R8 H, CH₃, CF₃, CH₂OH;
R9 H, (C₁-C₄)-alkyl;
R10 H, (C₁-C₄)-alkyl; or
R9 and R10 form together with the N atom to which they are bonded a 3-9 membered ring system;
and the physiologically tolerated salts thereof.

2. A compound of the formula I as claimed in claim 1, wherein the meanings therein are
R1 aryl, (C₁-C₆)-alkyl, (C₂-C₆)-alkenyl, (C₁-C₆)-alkylene-aryl, heterocycle, (C₁-C₆)-alkylene-heterocycle, CF₃, OCF₃, CN, (CH₂)₁₋₆₋OH, O-(C₁-C₆)-alkyl, CO-(C₁-C₆)-alkyl, C(O)O-alkyl, COOH, CON(R9)(R10), where the aryl radicals and the heterocyclic radicals may be substituted one or more times by F, Cl, Br, (CH₂)₀₋₂OH, (C₁₋C₆)-alkyl, (C₂-C₆)-alkenyl, (C₂-C₆)-alkynyl, CF₃, OCF₃, N(R9)(R10), piperidinone, piperazine, piperazinone, N-(C₁-C₆-alkylene)-piperazine, N-(C₁-C₆-alkylene)piperazinone, morpholine, thiomorpholine, NO₂, CN, O-(C₁-C₆)-alkyl, S(O)₀₋₂-(C₁-C₆)-alkyl, SO₂-N(R9)(R10), CO-(C₁-C₆)-alkyl, -COOH, (C₁-C₆)-alkylene-COOH, -COO(C₁-C₆)-alkyl, (C₀-C₆)-alkylene -COO(C₁-C₆)-alkyl, C₃-C₁₀-cycloalkyl, phenyl, where these piperidinone, piperazine, piperazinone, N-(C₁-C₆-alkylene)piperazine, N-(C₁-C₆₋alkylene)piperazinone, morpholine, thiomorpholine and phenylrings may be substituted one or more times by F, Cl, Br, (CH₂)₀₋₂OH, COOH, CN, NO₂, -O-(C₁-C₆)-alkyl,-NH-O-(C₁-C₆)-alkyl, -(CO)-NH-O-(C₁-C₆)-alkylene-N(R9)(R10), -(CO)-(C₁-C₆)-alkyl, -(C₁-C₆)-alkyl, CF₃, OCF₃, N(R9)(R10);
R2 H, aryl, COOH, (C₁-C₆)-alkylene-COOH,-COO(C₁-C₆)-alkyl, (C₁₋C₆)-alkylene-COO(C₁-C₆)-alkyl; (C₁-C₆)-alkyl, (C₂-C₆)-alkenyl, (C₁₋C₆)-alkylene-aryl, heterocycle, (C₁-C₆)-alkylene-heterocycle, CF₃, OCF₃, CN, -(CH₂)₁₋₆-OH, O-(C₁-C₆)-alkyl, CO-(C₁-C₆)-alkyl, C(O)O-alkyl, COOH, CON(R9)(R10), where the aryl radicals and the heterocyclic radicals may be substituted one or more times by F, Cl, Br, (CH₂)₀₋₂OH, (C₁-C₆)-alkyl, (C₂-C₆)-alkenyl, (C₂-C₆)-alkynyl, CF₃, OCF₃, N(R9)(R10), piperidinone, piperazine, piperazinone, N-(C₁-C₆-alkylene)piperazine, N-(C₁-C₆-alkylene)piperazinone, morpholine, thiomorpholine, NO₂, CN, O-(C₁-C₆)-alkyl, S(O)₀₋₂-(C₁₋C₆)-alkyl, SO₂-N(R9)(R10), CO-(C₁-C₆)-alkyl, -COOH, (C₁-C₆)-alkylene-COOH, -COO(C₁-C₆)-alkyl, (C₁-C₆)-alkylene-COO(C₁₋C₆)-alkyl, C₃-C₁₀-cycloalkyl, phenyl;
R3 H, (C₁-C₆)-alkyl, (C₁-C₆)-alkylene-aryl, -C(O)-aryl, (C₁-C₆)-alkylene-heterocycle , CO-(C₁-C₆)-alkyl;
R4, R5 independently of one another H, F, Cl, Br, (C₁-C₆)-alkyl, CF₃, OCF₃, NO₂, N(R9)(R10), CN, O-(C₁-C₆)-alkyl, CO-(C₁-C₆)-alkyl, COOH, (C₁-C₆)-alkylene-COOH, -CON(R9)(R10), (C₁-C₆)-alkylene-CON(R9)(R10), COO(C₁-C₆)-alkyl, (C₁-C₆)-alkylene-COO(C₁-C₆)-alkyl, S(O)₀₋₂-(C₁-C₆)-alkyl, S(O)₂-N(R9)(R10), CH₂OH, CH₂OCH₃;
R6, R7 independently of one another H, F, Cl, Br, (C₁-C₆)-alkyl, cyclopropyl, tetrafluorocyclopropyl, difluorocyclopropyl; or
R6 and R7 together form the group =CH₂;
R8 H, CH₃, CF₃, CH₂OH;
R9 H, (C₁-C₄)-alkyl;
R10 H, (C₁-C₄)-alkyl; or
R9 and R10 form together with the N atom to which they are bonded a 3-9 membered ring system;
and the physiologically tolerated salts thereof.

3. A compound of the formula I as claimed in claim 1 or 2, wherein the meanings therein are
R1 phenyl, naphthyl, thionaphthyl, pyridyl, where phenyl, naphthyl, thionaphthyl and pyridyl may be substituted one or more times by F, Cl, Br, (CH₂)₀₋₂OH, (C₁-C₆)-alkyl, (C₂-C₆)-alkenyl, (C₂-C₆)-alkynyl, CF₃, OCF₃, N(R9)(R10), piperidinone, piperazine, piperazinone, N-(C₁-C₆-alkylene)piperazine, N-(C₁-C₆-alkylene)piperazinone, morpholine, thiomorpholine, NO₂, CN, O-(C₁-C₆)-alkyl, S(O)₀₋₂-(C₁₋C₆)-alkyl, SO₂-N(R9)(R10), CO-(C₁-C₆)-alkyl, COOH, (C₁-C₆)-alkylene-COOH, COO(C₁-C₆)-alkyl, (C₁-C₆)-alkylene-COO(C₁-C₆)-alkyl, C₃-C₁₀-cycloalkyl, phenyl, where these piperidinone, piperazine, piperazinone, N-(C₁-C₆)-alkylene)-piperazine, N-(C₁-C₆₋alkylene)piperazinone, morpholine, thiomorpholine and phenylrings may be substituted one or more times by F, Cl, Br, (CH₂)₀₋₂OH, COOH, CN, NO₂, -O-(C₁-C₆)-alkyl, -NH-O-(C₁-C₆)-alkyl, -(CO)-NH-O-(C₁-C₆)-alkylene-N(R9)(R10), -(CO)-(C₁-C₆)-alkyl, -(C₁-C₆)-alkyl, CF₃, OCF₃, N(R9)(R10);
R2 H, (C₁-C₆)-alkyl, COOH, (C₁-C₆)-alkylene-COOH, -COO(C₁-C₆)-alkyl, (C₁-C₆)-alkylene-COO(C₁-C₆)-alkyl;
R3 H, (C₁-C₆)-alkyl, (C₁-C₆)-alkylene-aryl, -C(O)-aryl, (C₁-C₆)-alkylene-heterocycle, CO-(C₁-C₆)-alkyl
R4, R5 H;
R6, R7 H;
R8 H;
R9 H, (C₁-C₄)-alkyl;
R10 H, (C₁-C₄)-alkyl;
and the physiologically tolerated salts thereof.

4. A compound of the formula I, as claimed in one or more of claims 1 to 3, wherein the meanings therein are
R1 phenyl, where phenyl may be substituted one or more times by F, Cl, Br, (CH₂)₀₋₂OH, (C₁-C₆)-alkyl, (C₂-C₆)-alkenyl, (C₂-C₆)-alkynyl, CF₃, OCF₃, N(R9)(R10), piperidinone, piperazine, piperazinone, N-(C₁-C₆₋alkylene)piperazine, N-(C₁-C₆-alkylene)piperazinone, morpholine, thiomorpholine, NO₂, CN, O-(C₁-C₆)-alkyl, S(O)₀₋₂-(C₁-C₆)-alkyl, SO₂₋N(R9)(R10), CO-(C₁-C₆)-alkyl, COOH, (C₁-C₆)-alkylene-COOH, COO(C₁-C₆)-alkyl, (C₁-C₆)-alkylene-COO(C₁-C₆)-alkyl, C₃-C₁₀₋cycloalkyl, phenyl where these piperidinone, piperazine, piperazinone, N-(C₁-C₆)-alkylene)piperazine, N-(C₁-C₆-alkylene)piperazinone, morpholine, thiomorpholine and phenylrings may be substituted one or more times by F, Cl, Br, (CH₂)₀₋₂OH, COOH, CN, NO₂, -O-(C₁-C₆)-alkyl, -NH-O-(C₁-C₆)-alkyl, -(CO)-NH-O-(C₁-C₆)-alkylene-N(R9)(R10), -(CO)-(C₁-C₆)-alkyl, -(C₁-C₆)-alkyl, CF₃, OCF₃, N(R9)(R10);
R2 H, (C₁-C₆)-alkyl, -C(O)O-(C₁-C₆)-alkyl, -(C₁-C₆)-alkylene-C(O)-(C₁-C₆)-alkyl, -COOH, -(C₁-C₆)-alkylene-COOH;
R3 H, (C₁-C₆)-alkyl, (C₁-C₆)-alkylene-aryl, -C(O)-aryl, (C₁-C₆)-alkylene-heterocycle, CO-(C₁-C₆)-alkyl;
R4, R5 H;
R6, R7 H;
R8 H;
R9 H;
R10 H;
and the physiologically tolerated salts thereof.

5. The use of the compounds as claimed in one or more of claims 1 to 4 for producing a medicament.

6. A medicament comprising one or more of the compounds as claimed in one or more of claims 1 to 4.

7. A medicament comprising one or more of the compounds as claimed in one or more of claims 1 to 4 and at least one other active ingredient.

8. A medicament as claimed in claim 7, wherein the other active ingredient comprises one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

9. The use of the compounds as claimed in one or more of claims 1 to 4 for producing a medicament for reducing blood glucose.

10. The use of the compounds as claimed in one or more of claims 1 to 4 for producing a medicament for the treatment of type II diabetes.

11. The use of the compounds as claimed in one or more of claims 1 to 4 for producing a medicament for the treatment of disturbances of lipid and carbohydrate metabolism.

12. The use of the compounds as claimed in one or more of claims 1 to 4 for producing a medicament for the treatment of arteriosclerotic manifestations.

13. The use of the compounds as claimed in one or more of claims 1 to 4 for producing a medicament for the treatment of insulin resistance.

14. A process for producing a medicament comprising one or more of the compounds as claimed in one or more of claims 1 to 4, which comprises mixing the active ingredient with a pharmaceutically suitable carrier and converting this mixture into a form suitable for administration.

## Revendications

1. Composés de formule I, 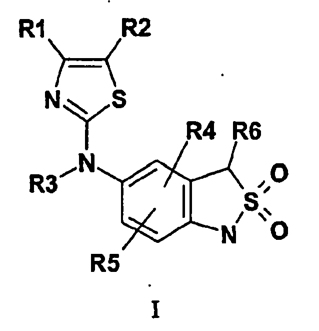 dans laquelle
R1, R2 signifient, indépendamment l'un de l'autre H, aryle, COOH, (C₁-C₆)-alkylène-COOH, -COO(C₁-C₆)-alkyle, (C₁-C₆)-alkylène-COO(C₁-C₆)-alkyle, (C₁-C₆)-alkyle, (C₂₋C₆)-alcényle, (C₁-C₆)-alkylène-aryle, hétérocycle, (C₁₋C₆)-alkylène-hétérocycle, CF₃, OCF₃, CN, (CH₂)₁₋₆-OH, O-(C₁-C₆)-alkyle, CO- (C₁-C₆)-alkyle, -C(O)O-alkyle, COOH, CON(R9)(R10), les radicaux aryle ainsi que les radicaux hétérocycliques pouvant être monosubstitués ou polysubstitués par F, Cl, Br, (CH₂)₀₋₂OH, (C₁-C₆)-alkyle, (C₂-C₆)-alcényle, (C₂-C₆)-alcynyle, CF₃, OCF₃, N(R9)(R10), pipéridinone, pipérazine, pipérazinone, N-(C₁-C₆-alkylène) -pipérazine, N- (C₁-C₆-alkylène) - pipérazinone, morpholine, thiomorpholine, NO₂, CN, O-(C₁-C₆)-alkyle, S (O)₀₋₂-(C₁-C₆)-alkyle, SO₂-N(R9)(R10), CO-(C₁-C₆)-alkyle, -COOH, (C₁-C₆)-alkylène-COOH, COO(C₁₋C₆)-alkyle, (C₁-C₆)-alkylène-COO(C₁-C₆)-alkyle, (C₃-C₁₀)-cycloalkyle, phényle, ces radicaux pipéridinone, pipérazine, pipérazinone, N-(C₁-C₆-alkylène)-pipérazine, N-(C₁-C₆-alkylène)-pipérazinone, morpholine, thiomorpholine et cycles phényle pouvant être monosubstitués ou polysubstitués par F, Cl, Br, (CH₂)₀₋₂OH, COOH, CN, NO₂, -O-(C₁-C₆)-alkyle, -NH-O-(C₁₋C₆)-alkyle, - (CO) -NH-O- (C₁-C₆)-alkylène-N(R9) (R10), - (CO) - (C₁-C₆)-alkyle, -(C₁-C₆)-alkyle, CF₃, OCF₃, N(R9)(R10) ;
R3 signifie H, (C₁-C₆) -alkyle, (C₁-C₆)-alkylène-aryle, - C(O)-aryle, (C₁-C₆)-alkylène-hétérocycle, CO- (C₁-C₆)-alkyle, les radicaux aryle et hétérocycliques pouvant être monosubstitués ou polysubstitués par F, Cl, Br, (C₁-C₆)-alkyle, COOH, COO(C₁-C₆)-alkyle, CF₃ ou OCF₃ ;
R4, R5 signifient indépendamment l'un de l'autre H, F, Cl, Br, (C₁-C₆)-alkyle, CF₃, OCF₃, NO₂, N(R9)(R10), CN, O-(C₁-C₆)-alkyle, CO- (C₁-C₆)-alkyle, COOH, (C₁-C₆)-alkylène-COOH, CON(R9)(R10), (C₁-C₆)-alkylène-CON(R9)(R10), COO(C₁-C₆)-alkyle, (C₁-C₆)-alkylène-COO(C₁₋C₆)-alkyle, S (O)₀₋₂-(C₁-C₆)-alkyle, S(O)₂-N(R9) (R10), CH₂OH, CH₂OCH₃ ;
R6, R7 signifient indépendamment l'un de l'autre H, F, Cl, Br, (C₁-C₆) -alkyle, cyclopropyle, tétrafluorocyclopropyle, difluorocyclopropyle ; ou R6 et R7 forment ensemble le groupe =CH₂ ;
R8 signifie H, CH₃, CF₃, CH₂OH ;
R9 signifie H, (C₁-C₄) -alkyle ;
R10 signifie H, (C₁-C₄)-alkyle ; ou
R9 et R10 forment ensemble avec l'atome de N auquel ils sont liés un système cyclique de 3-9 chaînons ;
ainsi que leurs sels physiologiquement acceptables.

2. Composés de formule I selon la revendication 1,
**caractérisés en ce que**
R1 signifie aryle, (C₁-C₆)-alkyle, (C₂-C₆)-alcényle, (C₁₋C₆)-alkylène-aryle, hétérocycle, (C₁-C₆)-alkylène-hétérocycle, CF₃, OCF₃, CN, (CH₂)₁₋₆-OH, O-(C₁-C₆)-alkyle, CO-(C₁-C₆)-alkyle, C(O)O-alkyle, COOH, CON(R9)(R10), les radicaux aryle ainsi que les radicaux hétérocycliques pouvant être monosubstitués ou polysubstitués par F, Cl, Br, (CH₂)₀₋₂OH, (C₁-C₆) -alkyle, (C₂-C₆) -alcényle, (C₂₋C₆)-alcynyle, CF₃, OCF₃, N(R9)(R10), pipéridinone, pipérazine, pipérazinone, N-(C₁-C₆-alkylène)-pipérazine, N-(C₁-C₆-alkylène)-pipérazinone, morpholine, thiomorpholine, NO₂, CN, O-(C₁-C₆)-alkyle, S(O)₀₋₂-(C₁₋C₆)-alkyle, SO₂-N(R9)(R10), CO-(C₁-C₆)-alkyle, -COOH, (C₁-C₆)-alkylène-COOH, -COO(C₁-C₆)-alkyle, (C₀-C₆)-alkylène-COO(C₁-C₆)-alkyle, C₃-C₁₀-cycloalkyle, phényle, ces radicaux pipéridinone, pipérazine, pipérazinone, N-(C₁-C₆-alkylène) -pipérazine, N- (C₁-C₆-alkylène) - pipérazinone, morpholine, thiomorpholine et cycles phényle pouvant être monosubstitués ou polysubstitués par F, Cl, Br, (CH₂)₀₋₂OH, COOH, CN, NO₂, -O-(C₁-C₆)-alkyle, -NH-O- (C₁-C₆) -alkyle, - (CO) -NH-O- (C₁-C₆) - alkylène-N(R9) (R10), - (CO) - (C₁-C₆) -alkyle, - (C₁-C₆)-alkyle, CF₃, OCF₃, N(R9)(R10) ;
R2 signifie H, aryle, COOH, (C₁-C₆)-alkylène-COOH, - COO (C₁-C₆) -alkyle, (C₁-C₆) -alkylène-COO (C₁-C₆) -alkyle, (C₁-C₆)-alkyle, (C₂-C₆)-alcényle, (C₁-C₆) -alkylène-aryle, hétérocycle, (C₁-C₆) -alkylène-hétérocycle, CF₃, OCF₃, CN, -(CH₂)₁₋₆-OH, O-(C₁-C₆)-alkyle, CO-(C₁-C₆)-alkyle, C(O)O-alkyle, COOH, CON(R9)(R10), les radicaux aryle ainsi que les radicaux hétérocycliques pouvant être monosubstitués ou polysubstitués par F, Cl, Br, (CH₂)₀₋₂OH, (C₁-C₆)-alkyle, (C₂-C₆)-alcényle, (C₂-C₆)-alcynyle, CF₃, OCF₃, N(R9)(R10), pipéridinone, pipérazine, pipérazinone, N-(C₁-C₆-alkylène)-pipérazine, N-(C₁-C₆-alkylène)-pipérazinone, morpholine, thiomorpholine, NO₂, CN, O-(C₁-C₆)-alkyle, S(O)₀₋₂-(C₁₋C₆) -alkyle, SO₂-N(R9) (R10), CO-(C₁-C₆)-alkyle, -COOH, (C₁-C₆) -alkylène-COOH, -COO (C₁-C₆) -alkyle, (C₁-C₆) - alkylène-COO(C₁-C₆)-alkyle, C₃-C₁₀-cycloalkyle, phényle ;
R3 signifie H, (C₁-C₆) -alkyle, (C₁-C₆) -alkylène-aryle, -C(O)-aryle, (C₁-C₆)-alkylène-hétérocycle, CO-(C₁-C₆)-alkyle ;
R4, R5 signifient indépendamment l'un de l'autre H, F, Cl, Br, (C₁-C₆)-alkyle, CF₃, OCF₃, NO₂, N(R9)(R10), CN, O-(C₁-C₆)-alkyle, CO- (C₁-C₆)-alkyle, COOH, (C₁-C₆)-alkylène-COOH, -CON(R9)(R10), (C₁-C₆) -alkylène-CON (R9) (R10), COO(C₁-C₆) -alkyle, (C₁-C₆)-alkylène-COO(C₁₋C₆)-alkyle, S (O)₀₋₂-(C₁-C₆)-alkyle, S(O)₂-N(R9) (R10), CH₂OH, CH₂OCH₃ ;
R6, R7 signifient indépendamment l'un de l'autre H, F, Cl, Br, (C₁-C₆)-alkyle, cyclopropyle, tétrafluorocyclopropyle, difluorocyclopropyle ; ou R6 et R7 forment ensemble le groupe =CH₂ ;
R8 signifie H, CH₃, CF₃, CH₂OH ;
R9 signifie H, (C₁-C₄)-alkyle ;
R10 signifie H, (C₁-C₄)-alkyle ; ou
R9 et R10 forment ensemble avec l'atome de N auquel ils sont liés un système cyclique de 3-9 chaînons ;
ainsi que leurs sels physiologiquement acceptables.

3. Composés de formule I selon la revendication 1 ou 2, **caractérisés en ce que**
R1 signifie phényle, naphtyle, thionaphtyle, pyridyle, où phényle, naphtyle, thionaphtyle et pyridyle peuvent être monosubstitués ou polysubstitués par F, Cl, Br, (CH₂)₀₋₂OH, (C₁-C₆) -alkyle, (C₂-C₆) -alcényle, (C₂-C₆) - alcynyle, CF₃, OCF₃, N(R9)(R10), pipéridinone, pipérazine, pipérazinone, N-(C₁-C₆-alkylène)-pipérazine, N-(C₁-C₆-alkylène)-pipérazinone, morpholine, thiomorpholine, NO₂, CN, O-(C₁-C₆)-alkyle, S(O)₀₋₂-(C₁₋C₆)-alkyle, SO₂-N(R9)(R10), CO-(C₁-C₆)-alkyle, COOH, (C₁₋C₆)-alkylène-COOH, COO(C₁-C₆)-alkyle, (C₁-C₆)-alkylène-COO(C₁-C₆)-alkyle, C₃-C₁₀-cycloalkyle, phényle, ces radicaux pipéridinone, pipérazine, pipérazinone, N-(C₁₋C₆-alkylène)-pipérazine, N-(C₁-C₆-alkylène)-pipérazinone, morpholine, thiomorpholine et cycles phényle pouvant être monosubstitués ou polysubstitués par F, Cl, Br, (CH₂)₀₋₂OH, COOH, CN, NO₂, -O-(C₁-C₆)-alkyle, -NH-O- (C₁-C₆)-alkyle, - (CO) -NH-O- (C₁-C₆)-alkylène-N(R9)(R10), - (CO) - (C₁-C₆)-alkyle, -(C₁-C₆)-alkyle, CF₃, OCF₃, N(R9) (R10) ;
R2 signifie H, (C₁-C₆)-alkyle, COOH, (C₁-C₆)-alkylène-COOH, -COO (C₁-C₆)-alkyle, (C₁-C₆)-alkylène-COO (C₁-C₆)-alkyle ;
R3 signifie H, (C₁-C₆)-alkyle, (C₁-C₆)-alkylène-aryle, -C(O)-aryle, (C₁-C₆)-alkylène hétérocycle, CO-(C₁-C₆)-alkyle ;
R4, R5 signifient H ;
R6, R7 signifient H ;
R8 signifie H ;
R9 signifie H, (C₁-C₄)-alkyle ;
R10 signifie H, (C₁-C₄)-alkyle ;
ainsi que leurs sels physiologiquement acceptables.

4. Composés de formule I selon l'une ou plusieurs des revendications 1 à 3, **caractérisés en ce que**
R1 signifie phényle, phényle pouvant être monosubstitué ou polysubstitué par F, Cl, Br, (CH₂)₀₋₂OH, (C₁-C₆) - alkyle, (C₂-C₆) -alcényle, (C₂-C₆) -alcynyle, CF₃, OCF₃, N(R9)(R10), pipéridinone, pipérazine, pipérazinone, N-(C₁-C₆-alkylène) -pipérazine, N-(C₁-C₆-alkylène) - pipérazinone, morpholine, thiomorpholine, NO₂, CN, O-(C₁-C₆)-alkyle, S(O)₀₋₂- (C₁-C₆) -alkyle, SO₂-N(R9) (R10), CO-(C₁-C₆)-alkyle, COOH, (C₁-C₆)-alkylène-COOH, COO(C₁₋C₆)-alkyle, (C₁-C₆) -alkylène-COO(C₁-C₆) -alkyle, C₃-C₁₀₋cycloalkyle, phényle, ces radicaux pipéridinone, pipérazine, pipérazinone, N-(C₁-C₆-alkylène)-pipérazine, N-(C₁-C₆-alkylène)-pipérazinone, morpholine, thiomorpholine et cycles phényle pouvant être monosubstitués ou polysubstitués par F, Cl, Br, (CH₂)₀₋₂₋OH, COOH, CN, NO₂, -O-(C₁-C₆)-alkyle, -NH-O-(C₁-C₆)-alkyle, - (CO) -NH-O- (C₁-C₆) -alkylène-N(R9) (R10), -(CO)-(C₁-C₆)-alkyle, - (C₁-C₆)-alkyle, CF₃, OCF₃, N(R9)(R10) ;
R2 signifie H, (C₁-C₆)-alkyle, -C(O)O-(C₁-C₆)-alkyle, - (C₁-C₆) -alkylène-C (O) O- (C₁-C₆) -alkyle, -COOH, - (C₁-C₆) - alkylène-COOH ;
R3 signifie H, (C₁-C₆)-alkyle, (C₁-C₆)-alkylène-aryle, - C(O)-aryle, (C₁-C₆) -alkylène-hétérocycle, CO- (C₁-C₆) - alkyle ;
R4, R5 signifient H ;
R6, R7 signifient H ;
R8 signifie H ;
R9 signifie H
R10 signifie H
ainsi que leurs sels physiologiquement acceptables.

5. Utilisation des composés selon l'une ou plusieurs des revendications 1 à 4 pour la préparation d'un médicament.

6. Médicament contenant un ou plusieurs des composés selon l'une ou plusieurs des revendications 1 à 4.

7. Médicament contenant un ou plusieurs des composés selon l'une ou plusieurs des revendications 1 à 4 et au moins une autre substance active.

8. Médicament selon la revendication 7,
**caractérisé en ce qu'**il contient comme autre substance active un ou plusieurs agents antidiabétiques, une ou plusieurs substances actives hypoglycémiques, un ou plusieurs inhibiteurs de la HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption de l'acide gallique, inhibiteurs de CETP, adsorbants polymères de l'acide gallique, inducteurs du récepteur de LDL, inhibiteurs d'ACAT, antioxydants, inhibiteurs de la lipoprotéine-lipase, inhibiteurs de l'ATP-citrate-lyase, inhibiteurs de la squalène synthétase, antagonistes de la lipoprotéine (a), inhibiteurs de lipase, l'insuline, une ou plusieurs sulfonylurées, un ou plusieurs biguanides, méglitinides, une ou plusieurs thiazolidinediones, un ou plusieurs inhibiteurs de l'α-glucosidase, une ou plusieurs substances actives agissant sur le canal potassique des cellules bêta dépendant de l'ATP, un ou plusieurs agonistes de CART, agonistes de NPY, agonistes de MC4, agonistes d'orexine, agonistes de H3, agonistes de TNF, agonistes de CRF, agonistes de CRF BP, agonistes de l'urocortine, agonistes de β3, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, une ou plusieurs hormones de croissance, un ou plusieurs composés libérant une hormone de croissance, agonistes de TRH, modulateurs 2 ou 3 découplant les protéines, agonistes de leptine, agonistes de DA (bromocriptine, doprexine), inhibiteurs de lipase/amylase, modulateurs de PPAR, modulateurs de RXR ou agonistes de TR-β ou amphétamines.

9. Utilisation des composés selon l'une ou plusieurs des revendications 1 à 4 pour la préparation d'un médicament destiné à abaisser la glycémie.

10. Utilisation des composés selon l'une ou plusieurs des revendications 1 à 4 pour la préparation d'un médicament destiné au traitement du diabète de type II.

11. Utilisation des composés selon l'une ou plusieurs des revendications 1 à 4 pour la préparation d'un médicament destiné au traitement des troubles du métabolisme lipidique et des hydrates de carbone.

12. Utilisation des composés selon l'une ou plusieurs des revendications 1 à 4 pour la préparation d'un médicament destiné au traitement de phénomènes artérioscléreux.

13. Utilisation des composés selon l'une ou plusieurs des revendications 1 à 4 pour la préparation d'un médicament destiné au traitement de la résistance à l'insuline.

14. Procédé pour la préparation d'un médicament contenant un ou plusieurs composés selon l'une ou plusieurs des revendications 1 à 4, **caractérisé en ce que** la substance active est mélangée avec un support pharmaceutiquement acceptable et ce mélange est amené dans une forme appropriée pour l'administration.
